(19) **Europäisches Patentamt / European Patent Office / Office européen des brevets**

(11) **EP 1 585 987 B1**

(12) **EUROPEAN PATENT SPECIFICATION**

(45) Date of publication and mention
of the grant of the patent:
**19.08.2009 Bulletin 2009/34**

(51) Int Cl.:
**G01N 33/574** [(2006.01)]

(21) Application number: **04701189.5**

(86) International application number:
**PCT/US2004/000410**

(22) Date of filing: **09.01.2004**

(87) International publication number:
**WO 2004/063711 (29.07.2004 Gazette 2004/31)**

(54) **USE OF ADIPONECTIN TO DIAGNOSE MALIGNANCY**

VERWENDUNG VON ADIPONECTIN ZUR DIAGNOSE VON KREBS

UTILISATION DE L'ADIPONECTINE POUR DIAGNOSTIQUER UNE MANIFESTATION MALIGNE

(84) Designated Contracting States:
**AT BE BG CH CY CZ DE DK EE ES FI FR GB GR
HU IE IT LI LU MC NL PT RO SE SI SK TR**

(30) Priority: **09.01.2003 US 439088 P**

(43) Date of publication of application:
**19.10.2005 Bulletin 2005/42**

(73) Proprietor: **Beth Israel Deaconess Medical Center,
Inc.
Boston, MA 02215 (US)**

(72) Inventor: **MANTZOROS, Christos, S.
Brookline, MA 02445 (US)**

(74) Representative: **Kirkham, Nicholas Andrew et al
Graham Watt & Co LLP
St Botolph's House
7-9 St Botolph's Road
Sevenoaks
Kent TN13 3AJ (GB)**

(56) References cited:
**WO-A-99/02546          WO-A-99/07736
WO-A-02/061076**

• **PETRIDOU E ET AL: "Clinical case seminar -
Plasma adiponectin concentrations in relation to
endometrial cancer: A case-control study in
Greece" JOURNAL OF CLINICAL
ENDOCRINOLOGY AND METABOLISM 01 MAR
2003 UNITED STATES, vol. 88, no. 3, 1 March 2003
(2003-03-01), pages 993-997, XP002279452 ISSN:
0021-972X**

**Description**

BACKGROUND OF THE INVENTION

EP 1 365 022 discloses an adiponectin-associated protein, WO99/02546 discloses human secreted proteins.

Endometrial cancer is the most common pelvic gynecologic malignancy. An increased incidence of endometrial cancer has been associated with prolonged, unopposed estrogen exposure (Ziel, H.K. and Finkle, W.D., New Engl. J. Med 1975; 293(23): 1167-1170; Jick, S.S. et al., Epidemiology 1993; 4(1): 20-24); however, combination therapy with estrogen and progesterone prevents the increase in risk of endometrial cancer associated with unopposed estrogen use (Jick, S.S., Epidemiology 1993; 4(4): 384; Bilezikian, J.P., Journal of Women's Health 1994; 3(4): 273-282). An increase in the incidence of endometrial cancer has also been associated with tamoxifen treatment of breast cancer, perhaps related to the estrogenic effect of tamoxifen on the endometrium (van Leeuwen, F.E. et al., Lancet 1994; 343 (8895): 448-452; Fisher, B. et al., Journal of the National Cancer Institute 1994; 86(7): 527-537).

Endometrial cancer has consistently been associated with obesity, which is currently accepted as a risk factor for the development of the disease (Rose, P.G., N. Engl. J. Med. 1996; 335(9):640-649). It is hypothesized that adipose tissue serves as the site of peripheral aromatization of the circulating adrenal androgen androstenedione to estrone. This increase in endogenous estrogen production acts as an agonist of endometrial cell growth (Judd, H.L. et al., Obstet Gynecol 1982; 59:680-6; Deslypere, J.P., Metabolism 1995; 44:24-27; Carroll, K.K., Lipids 1998; 33:1055-1059). Another link between endometrial cancer and obesity may be insulin resistance (Parazzini, F. et al., Int J. Cancer 1999; 81(4): 539-42). It remains unknown what the underlying etiologic factor is and whether this putative factor leads to changes in insulin resistance or estrogen levels.

SUMMARY OF THE INVENTION

The present invention pertains to methods of diagnosing the presence or absence of endometrial cancer or a risk of endometrial cancer in an individual (e.g., a woman who is less than 65 years of age), in which a test sample from the woman is assessed for a level of adiponectin. In one embodiment, the level of adiponectin is compared to a reference level; the presence of a level of adiponectin that is equal to or less than a reference level is indicative of the presence of endometrial cancer, and the presence of a level of adiponectin that is greater than a reference level is indicative of the absence of endometrial cancer. In another embodiment, the level of adiponectin is compared to a control level; the presence of a level of adiponectin that is less than the control level, by an amount that is statistically significant, is indicative of the presence of endometrial cancer, and the presence of a level of adiponectin that is greater than the control level, by an amount that is statistically significant, or is equal to the control level, is indicative of the absence of endometrial cancer. In a further embodiment, the level of adiponectin is compared to a level of adiponectin in at least one comparable negative control sample; the presence of a level of adiponectin that is less than a level of adiponectin in a comparable negative control sample, by an amount that is statistically significant, is indicative of the presence of endometrial cancer, and the presence of a level of adiponectin that is greater than a level of adiponectin in a comparable negative control sample, by an amount that is statistically significant, or is equal to a level of adiponectin in a comparable negative control sample, is indicative of the absence of endometrial cancer.

In an additional embodiment, the level of adiponectin is compared to a reference level; the presence of a level of adiponectin that is equal to or less than a reference level is indicative of the presence of a risk of endometrial cancer, and the presence of a level of adiponectin that is greater than a reference level is indicative of the absence of a risk of endometrial cancer. In another embodiment, the level of adiponectin is compared to a control level; the presence of a level of adiponectin that is less than the control level, by an amount that is statistically significant, is indicative of the presence of a risk of endometrial cancer, and the presence of a level of adiponectin that is greater than the control level, by an amount that is statistically significant, or is equal to the control level, is indicative of the absence of a risk of endometrial cancer. In a further embodiment, the level of adiponectin is compared to a level of adiponectin in at least one comparable negative control sample; the presence of a level of adiponectin that is less than a level of adiponectin in a comparable negative control sample, by an amount that is statistically significant, is indicative of the pretence of a risk of endometrial cancer, and the presence of a level of adiponectin that is greater than a level of adiponectin in a comparable negative control sample, by an amount that is statistically significant, or is equal to a level of adiponectin in a comparable negative control sample, is indicative of the absence of a risk of endometrial cancer.

Methods of treating endometrial cancer in an individual are disclosed, by administering adiponectin, the globular domain, and/or both adiponectin and the globular domain, and/or an adiponectin receptor agonist and/or another adiponectin therapeutic agent (e.g., in a pharmaceutical composition) to the individual in a therapeutically effective amount.

The present invention additionally pertains to methods of diagnosing the presence or absence of an epithelial cancer or a risk of an epithelial cancer in an individual, in which a test sample from the individual is assessed for a level of adiponectin. Representative epithelial cancers include breast cancer, ovarian cancer, prostate cancer, leukemia, and colon cancer. In a preferred embodiment, the epithelial cancer is breast cancer, and the individual is a post-menopausal woman.

In one embodiment, the level of adiponectin is compared to a reference level; the presence of a level of adiponectin that is equal to or less than a reference level is indicative of the presence of the epithelial cancer, and the presence of a level of adiponectin that is greater than a reference level is indicative of the absence of the epithelial cancer. In another embodiment, the level of adiponectin is compared to a control level; the presence of a level of adiponectin that is less than the control level, by an amount that is statistically significant, is indicative of the presence of the epithelial cancer, and the presence of a level of adiponectin that is greater than the control level, by an amount that is statistically significant, or is equal to the control level, is indicative of the absence of the epithelial cancer. In a further embodiment, the level of adiponectin is compared to a level of adiponectin in at least one comparable negative control sample; the presence of a level of adiponectin that is less than a level of adiponectin in a comparable negative control sample, by an amount that is statistically significant, is indicative of the presence of the epithelial cancer, and the presence of a level of adiponectin that is greater than a level of adiponectin in a comparable negative control sample, by an amount that is statistically significant, or is equal to a level of adiponectin in a comparable negative control sample, is indicative of the absence of the epithelial cancer.

In an additional embodiment, the level of adiponectin is compared to a reference level; the presence of a level of adiponectin that is equal to or less than a reference level is indicative of the presence of a risk of the epithelial cancer, and the presence of a level of adiponectin that is greater than a reference level is indicative of the absence of a risk of the epithelial cancer. In another embodiment, the level of adiponectin is compared to a control level; the presence of a level of adiponectin that is less than the control level, by an amount that is statistically significant, is indicative of the presence of a risk of the epithelial cancer, and the presence of a level of adiponectin that is greater than the control level, by an amount that is statistically significant, or is equal to the control level, is indicative of the absence of a risk of the epithelial cancer. In a further embodiment, the level of adiponectin is compared to a level of adiponectin in at least one comparable negative control sample; the presence of a level of adiponectin that is less than a level of adiponectin in a comparable negative control sample, by an amount that is statistically significant, is indicative of the presence of a risk of the epithelial cancer, and the presence of a level of adiponectin that is greater than a level of adiponectin in a comparable negative control sample, by an amount that is statistically significant, or is equal to a level of adiponectin in a comparable negative control sample, is indicative of the absence of a risk of the epithelial cancer.

Methods of treating an epithelial cancer in an individual are disclosed, by administering adiponectin, the globular domain of adiponectin, and/or a combination of adiponectin and the globular domain of adiponectin, and/or an adiponectin receptor agonist, and/or another adiponectin therapeutic agent (e.g., in a pharmaceutical composition) to the individual in a therapeutically effective amount.

The present invention also pertains to methods of diagnosing the presence or absence of a risk of relapse of an epithelial cancer in an individual, or determining the survival from the specific malignancy, in which a test sample from the individual is assessed for a level of adiponectin. In one embodiment, the level of adiponectin is compared to a reference level; the presence of a level of adiponectin that is equal to or less than a reference level is indicative of the presence of a risk of relapse the epithelial cancer, and the presence of a level of adiponectin that is greater than a reference level is indicative of the absence of a risk of relapse of the epithelial cancer. In another embodiment, the level of adiponectin is compared to a control level; the presence of a level of adiponectin that is less than the control level, by an amount that is statistically significant, is indicative of the presence of a risk of relapse of the epithelial cancer, and the presence of a level of adiponectin that is greater than the control level, by an amount that is statistically significant, or is equal to the control level, is indicative of the absence of a risk of relapse of the epithelial cancer. In a further embodiment, the level of adiponectin is compared to a level of adiponectin in at least one comparable negative control sample; the presence of a level of adiponectin that is less than a level of adiponectin in a comparable negative control sample, by an amount that is statistically significant, is indicative of the presence of a risk of relapse the epithelial cancer, and the presence of a level of adiponectin that is greater than a level of adiponectin in a comparable negative control sample, by an amount that is statistically significant, or is equal to a level of adiponectin in a comparable negative control sample, is indicative of the absence of a risk of relapse the epithelial cancer.

DETAILED DESCRIPTION OF THE INVENTION

The present invention pertains to methods for the diagnosis of certain cancers or of a risk of certain cancers, such as endometrial cancer. As described herein, Applicant has discovered that the level of adiponectin in a sample from an individual correlates inversely with the presence of endometrial cancer in women younger than 65 years of age. This discovery has enabled the development of methods of diagnosis and treatment of certain types of cancers, as well as the development and manufacture of medicaments for the treatments of these cancers.

Adiponectin (acrp30, adipoQ, *apM1* gene product) is a recently discovered protein which is secreted exclusively by adipocytes (Scherer, P.E. et al., J Biol Chem 1995; 270:26746-26749; Nakano, Y. et al., J Biochem 1996; 120:

803-812; Hu, E. et al., J Biol Chem 1996; 271:10697-10703; Maeda, K. et al., Biochem Biophys Res Commun 1996; 221:286-289). Although secreted only by adipose tissue, adiponectin levels are paradoxically decreased in obesity and type 2 diabetes mellitus, conditions often associated with insulin resistance (Hu, E. et al., J Biol Chem 1996; 271: 10697-10703; Arita, Y. et al., Biochem Biophy Res Commun 1999; 257:79-83; Weyer, C. et al., J Clin Endocrinol Metab 2001; 86:1930-1935; Hotta, K. et al., Arterioscler Thromb Vasc Biol 2000). Applicant has discovered that, in women younger than 65 years, adiponectin is inversely and significantly related to the risk of endometrial cancer, and the association is independent of possible effects of major components of the insulin like growth system, leptin and gynaecological parameters. In addition, applicant has discovered that there is an inverse, fairly strong and statistically significant association of serum adiponectin with breast cancer in postmenopausal women.

*Methods of Diagnosis: Endometrial Cancer*

As a result of this discovery, in one embodiment of the invention, methods are now available for diagnosing endometrial cancer or a risk of endometrial cancer. The methods diagnose the presence or absence of endometrial cancer or of a risk of endometrial cancer, by assessing a test sample from an individual for the level of adiponectin in the sample. The level of adiponectin is inversely correlated with endometrial cancer or a risk of endometrial cancer.

As used herein, the term "endometrial cancer" refers to a malignancy that arises from the inner lining of the uterus (endometrium). The term, "risk of endometrial cancer" as used herein, refers to an adiponectin-associated risk of endometrial cancer. While other risk factors exist for endometrial cancer, the methods described herein pertain to risk associated with levels of adiponectin.

In the methods of the invention, a "test sample" from an individual to be assessed for endometrial cancer or for risk of endometrial cancer is used. The test sample can comprise blood, serum, cerebrospinal fluid, urine, nasal secretion, saliva, or any other bodily fluid or tissue. In a preferred embodiment, the test sample is a blood or serum sample from the individual. In a preferred embodiment, the individual to be assessed for endometrial cancer or for risk of endometrial cancer is a woman who is less than 65 years of age.

The level of adiponectin in the test sample is then measured, using standard methods, such as by enzyme-linked immunosorbent assay (ELISA).

In one embodiment of the invention, the level of adiponectin is compared to a reference level. The term, "reference level," as used herein, refers to a level or amount of adiponectin that correlates with a diagnosis of endometrial cancer, and/or with a risk of endometrial cancer. A reference level can be determined, for example, by comparing levels of adiponectin in samples from individuals known to have endometrial cancer, with levels of adiponectin in samples from individuals known not to have endometrial cancer (e.g., a "negative control sample" as described below and in the Exemplification), and determining what level of adiponectin correlates with disease or with risk of diease. The reference level can be determined by determining the level of adiponectin in positive and/or negative control samples concurrently with determining the level of adiponectin in the test sample; alternatively, the reference level can be a historically determined level (i.e., a level determined prior to determining the level of adiponectin in the test sample). For example, in one embodiment, a "reference level" can be a level of adiponectin in the test sample that statistically is significantly less than the level of adiponectin in comparable control sample(s), such as an amount that is at least about about two standard deviations below, or about three or more standard deviations below, the level of adiponectin in comparable control samples. For example, in another embodiment, the "reference level" can be one quintile below the level of adiponectin in comparable control sample(s).

In this embodiment, the presence of a level that is equal to, or less than, the reference level correlates with a diagnosis of (is indicative of the presence of) endometrial cancer and/or a risk of endometrial cancer. A level that that is greater than the reference level correlates with (is indicative of) an absence of a diagnosis of endometrial cancer and/or a risk of endometrial cancer.

In another embodiment of the invention, the level of adiponectin is compared to a control level. The term, "control level," as used herein, refers to a level or amount of adiponectin that correlates with an absence of endometrial cancer. A control level can be determined, for example, by assessing levels of adiponectin in samples from individuals known not to have endometrial cancer (e.g., a "negative control sample" as described below and in the Exemplification) or another epithelial cancer. The control level can be determined by determining the level of adiponectin in negative control samples concurrently with determining the level of adiponectin in the test sample, as described below; alternatively, the control level can be a historically determined level (i.e., a level determined prior to determining the level of adiponectin in the test sample). For example, in one embodiment, a "control level" can be a level of adiponectin in a test sample of serum, that is about 13.53 $\mu$g/mL $\pm$ 5.26 $\mu$g/mL, as described in the Exemplification.

In this embodiment, the presence of a level that is less than the control level by an amount that is statistically significant, correlates with a diagnosis of (is indicative of the presence of) endometrial cancer and/or a risk of endometrial cancer. A level that that is equal to or greater than the control level, by an amount that is statistically significant correlates with (is indicative of) an absence of a diagnosis of endometrial cancer and/or a risk of endometrial cancer. For example, a "statistically significant" difference can be a level of adiponectin in the test sample that is significantly less than the level of adiponectin in comparable control sample(s), such as an amount that is at least about about two standard deviations below, or about three or more standard deviations below, the level of adiponectin in comparable control samples. For example, in another embodiment, the difference can be statistically significant if the test level is one quintile below the control level.

In yet another embodiment of the invention, the test sample is assayed to determine the level of adiponectin, as above. The level of adiponectin in the test sample is compared with the level of adiponectin in at least one comparable negative control sample (i.e., a sample from an individual who is not affected by endometrial cancer). The negative control sample can be a sample from any individual who is not affected by endometrial cancer; it is not necessary that the negative control sample be from an individual who is free of disease. A "comparable" negative control sample is a sample of the same type of body fluid or tissue as the test sample. More than one control sample can be used.

In this embodiment, the presence of a level of adiponectin in the test sample that is significantly less than the level of adiponectin in a comparable control sample(s), as described above, correlates with the presence of endometrial cancer and/or a risk of endometrial cancer. The presence of a level of adiponectin in the test sample that is not significantly less than the level of adiponectin in a comparable control sample(s), correlates with an absence of endometrial cancer and/or a risk of endometrial cancer.

*Methods of Diagnosis: Other Cancers*

The methods described above with regard to endometrial cancer can be applied in a similar manner to other malignancies. A reference level, control level and/or level of adiponectin in a control sample can be determined as described herein. The level of adiponectin in a test sample from an individual can be assessed and compared to the reference level, control level, and/or level of adiponectin in a comparable control sample(s), and correlated by statistical significance to a presence or absence of disease and/or a presence or absence of an adiponectin-associated risk of disease, as described herein.

Representative malignancies include those with at least one etiology common with that of endometrial cancer: the malignancy is an epithelial cancer, is associated with abnormal sex steroid levels, and is related to obesity. In one preferred embodiment, the malignancy has the characteristic of being an epithelial cancer; in another preferred embodiment, the malignancy has all three characteristics (epithelial cancer, associated with abnormal sex steroid levels, and related to obesity). In particular embodiments, the malignancy can be ovarian cancer; prostate cancer, leukemia, or colon cancer. In another particular embodiment, the malignancy can be breast cancer, and the individual is a postmenopausal woman.

As used herein, the term "epithelial cancer" refers to a malignancy that arises from an epithelial layer of tissue. Representative epithelial cancers include endometrial, breast; ovarian, and prostate cancers. The term, "risk of epithelial cancer" as used herein, refers to an adiponectin-associated risk of epithelial cancer. While other risk factors may exist for the epithelial cancer, the methods described herein pertain to risk associated with levels of adiponectin.

In the methods of the invention, a "test sample" from an individual to be assessed for the epithelial cancer or for risk of the epithelial cancer is used; the test sample can comprise blood, serum, cerebrospinal fluid, urine, nasal secretion, saliva, or any other bodily fluid or tissue, as described above in relation to endothelial cancer. The level of adiponectin in the test sample is then measured, using standard methods, such as by enzyme-linked immunosorbent assay (ELISA). As above, the level of adiponectin is compared to a reference level. The term, "reference level," as used herein, refers to a level or amount of adiponectin that correlates with a diagnosis of that epithelial cancer, and/or with a risk of that epithelial cancer. A reference level can be determined, for example, by comparing levels of adiponectin in samples from individuals known to have that epithelial cancer, with levels of adiponectin in samples from individuals known not to have any epithelial cancer (e.g., a "negative control sample"), and determining what level of adiponectin correlates with disease or with risk of diease. The reference level can be determined by determining the level of adiponectin in positive and/or negative control samples concurrently with determining the level of adiponectin in the test sample; alternatively, the reference level can be a historically determined level (i.e., a level determined prior to determining the level of adiponectin in the test sample). For example, in one embodiment, a "reference level" can be a level of adiponectin in the test sample that statistically is significantly less than the level of adiponectin in comparable control sample(s), such as an amount that is at least about about two standard deviations below, or about three or more standard deviations below, the level of adiponectin in comparable control samples. For example, in another embodiment, the "reference level" can be one quintile below the level of adiponectin in comparable control sample(s).

In this embodiment, the presence of a level that is equal to, or less than, the reference level correlates with a diagnosis of (is indicative of the presence of) the epithelial cancer and/or a risk of epithelial cancer. A level that that is greater than the reference level correlates with (is indicative of) an absence of a diagnosis of epithelial cancer and/or a risk of epithelial cancer.

In another embodiment of the invention, the level of adiponectin is compared to a control level. The term, "control level," as used herein, refers to a level or amount of adiponectin that correlates with an absence of epithelial cancer. A control level can be determined, for example, by assessing levels of adiponectin in samples from individuals known not to have an epithelial cancer (e.g., a "negative control sample"). The control level can be determined by determining the level of adiponectin in negative control samples concurrently with determining the level of adiponectin in the test sample, as described below; alternatively, the control level can be a historically determined level (i.e., a level determined prior to determining the level of adiponectin in the test sample). In this embodiment, the presence of a level that is less than the control level by an amount that is statistically significant, correlates with a diagnosis of (is indicative of the presence of) epithelial cancer and/or a risk of epithelial cancer. A level that that is equal to or greater than the control level, by an amount that is statistically significant correlates with (is indicative of) an absence of a diagnosis of epithelial cancer and/or a risk of epithelial cancer. For example, a "statistically significant" difference can be a level of adiponectin in the test sample that is significantly less than the level of adiponectin in comparable control sample(s), such as an amount that is at least about one standard deviation below, or about two standard deviations below, or about three or more standard deviations below, the level of adiponectin in comparable control samples. For example, in another embodiment, the difference can be statistically significant if the test level is one quintile below the control level.

In yet another embodiment of the invention, the test sample is assayed to determine the level of adiponectin, as above. The level of adiponectin in the test sample is compared with the level of adiponectin in at least one comparable negative control sample (i.e., a sample from an individual who is not affected by epithelial cancer). The negative control sample can be a sample from any individual who is not affected by epithelial cancer; it is not necessary that the negative control sample be from an individual who is free of disease. A "comparable" negative control sample is a sample of the same type of body fluid or tissue as the test sample. More than one control sample can be used. In this embodiment, the presence of a level of adiponectin in the test sample that is significantly less than the level of adiponectin in a comparable control sample(s), as described above, correlates with the presence of epithelial cancer and/or a risk of epithelial cancer. The presence of a level of adiponectin in the test sample that is not significantly less than the level of adiponectin in a comparable control sample(s), correlates with an absence of epithelial cancer and/or a risk of epithelial cancer.

In using negative control samples, reference levels, or control levels, the relevant population should be considered. For example, for breast cancer, the relevant population is post-menopausal women; thus, control samples should be from that population.

*Methods of Diagnosis: Risk of Relapse*

The methods of diagnosis described above can be applied in a similar manner to assess an individual for a risk of relapse after treatment for an epithelial cancer. A "risk of relapse," as used herein, refers to an adiponectin-associated risk for the return of the epithelial cancer after treatment. While other risk factors may exist for relapse, the methods described herein pertain to risk associated with levels of adiponectin. Alternatively, the "risk of relapse" can be referred to as the survival rate from the specific malignancy: those with a low risk of relapse are expected to have a higher survival rate, and those with a high risk or relapse correlatively are expected to have a lower survival rate.

As described above, a reference level, control level and/or level of adiponectin in a control sample can be determined as described herein. The level of adiponectin in a test sample from an individual after treatment can be assessed and compared to the reference level, control level, and/or level of adiponectin in a comparable control sample(s), and correlated by statistical significance to a presence or absence of disease and/or a presence or absence of an adiponectin-associated risk of disease, as described herein. The presence of a level of adiponectin in the test sample that is significantly less than the reference level, control level, and/or level of adiponectin in a comparable control sample(s), as described above, correlates with the presence of an increased risk of relapse. The presence of a level of adiponectin in the test sample that is not significantly less, correlates with an absence of risk of relapse. Similarly, the presence of a level of adiponectin in the test sample that is significantly less than the reference level, control level, and/or level of adiponectin in a comparable control sample(s), as described above, correlates with the presence of a decreased survival rate. The presence of a level of adiponectin in the test sample that is not significantly less, correlates with an increased survival rate.

*Methods of Treatment* (not claimed)

In addition to the methods of diagosis, methods are now available for treatment for endometrial cancer and other epithelial cancers as described above, as are methods for the manufacture of a medicament for the treatment of such cancers. The term, "treatment" as used herein, can refer to ameliorating symptoms associated with the cancer, to preventing or delaying the onset of the cancer (e.g., in individuals suspected of being at risk for the cancer, or specifically identified as being at risk for the cancer, such as by the methods described above), to lessening the severity, duration or frequency of symptoms of the cancer, and/or to improving the survivial time of an individual having the cancer.

In one method of treatment, adiponectin is administered to the individual. The adiponectin can be administered as a complete molecule; alternatively, the globular domain that is the active part of adiponectin can be administered. If desired, a mixture of full-length adiponectin and the globular domain of adiponectin can be administered. For description of the globular domain, see, for example, see Hu, X.-B. et al., Acta Biochim. Biophys. Sin. 2003: 35(11):1023-1028; Fruebis, J. et al., PNAS USA 2001 98(4):2005-2010; Tomas, E. et al., PNAS USA 2002 99(25):16309-16313. As used herein, administration of "adiponectin" can include full-length adiponectin, the globular domain of adiponectin, or both.

Adiponectin can be administered or in a pharmaceutical composition. For example, adiponectin and/or an adiponectin receptor agonist can be formulated together with a physiologically acceptable carrier or excipient to prepare a pharmaceutical composition. The carrier and composition can be sterile. The formulation should suit the mode of administration.

Suitable pharmaceutically acceptable carriers include but are not limited to water, salt solutions (*e.g.*, NaCl), saline, buffered saline, alcohols, glycerol, ethanol, gum arabic, vegetable oils, benzyl alcohols, polyethylene glycols, gelatin, carbohydrates such as lactose, amylose or starch, dextrose, magnesium stearate, talc, silicic acid, viscous paraffin, perfume oil, fatty acid esters, hydroxymethylcellulose, polyvinyl pyrolidone, etc., as well as combinations thereof. The pharmaceutical preparations can, if desired, be mixed with auxiliary agents, *e.g.*, lubricants, preservatives, stabilizers, wetting agents, emulsifiers, salts for influencing osmotic pressure, buffers, coloring, flavoring and/or aromatic substances and the like which do not deleteriously redact with the active agents.

The composition, if desired, can also contain minor amounts of wetting or emulsifying agents, or pH buffering agents. The composition can be a liquid solution, suspension, emulsion, tablet, pill, capsule, sustained release formulation, or powder. The composition can be formulated as a suppository, with traditional binders and carriers such as triglycerides. Oral formulation can include standard carriers such as pharmaceutical grades of mannitol, lactose, starch, magnesium stearate, polyvinyl pyrollidone, sodium saccharine, cellulose, magnesium carbonate, etc.

Methods of introduction of these compositions include, but are not limited to, intradermal, intramuscular, intraperitoneal, intraocular, intravenous, subcutaneous, topical, oral and intranasal. Other suitable methods of introduction can also include gene therapy (e.g., administration of a nucleic acid encoding adiponectin), rechargeable or biodegradable devices, particle acceleration devises ("gene guns") and slow release polymeric devices. The pharmaceutical compositions can also be administered as part of a combinatorial therapy with other agents.

The composition can be formulated in accordance with the routine procedures as a pharmaceutical composition adapted for administration to human beings. For example, compositions for intravenous administration typically are solutions in sterile isotonic aqueous buffer. Where necessary, the composition may also include a solubilizing agent and a local anesthetic to ease pain at the site of the injection. Generally, the ingredients are supplied either separately or mixed together in unit dosage form, for example, as a dry lyophilized powder or water free concentrate in a hermetically sealed container such as an ampule or sachette indicating the quantity of active agent. Where the composition is to be administered by infusion, it can be dispensed with an infusion bottle containing sterile pharmaceutical grade water, saline or dextrose/water. Where the composition is administered by injection, an ampule of sterile water for injection or saline can be provided so that the ingredients may be mixed prior to administration.

For topical application, nonsprayable forms, viscous to semi-solid or solid forms comprising a carrier compatible with topical application and having a dynamic viscosity preferably greater than water, can be employed. Suitable formulations include but are not limited to solutions, suspensions, emulsions, creams, ointments, powders, enemas, lotions, sols, liniments, salves, aerosols, etc., which are, if desired, sterilized or mixed with auxiliary agents, *e.g.*, preservatives, stabilizers, wetting agents, buffers or salts for influencing osmotic pressure, etc. The agent may be incorporated into a cosmetic formulation. For topical application, also suitable are sprayable aerosol preparations wherein the active ingredient, preferably in combination with a solid or liquid inert carrier material, is packaged in a squeeze bottle or in admixture with a pressurized volatile, normally gaseous propellant, *e.g.*, pressurized air.

Agents described herein can be formulated as neutral or salt forms. Pharmaceutically acceptable salts include those formed with free amino groups such as those derived from hydrochloric, phosphoric, acetic, oxalic, tartaric acids, etc., and those formed with free carboxyl groups such as those derived from sodium, potassium, ammonium, calcium, ferric hydroxides, isopropylamine, triethylamine, 2-ethylamino ethanol, histidine, procaine, etc.

Adiponectin and/or an adiponectin receptor agonist, whether alone or in a pharmaceutical composition, is administered in a therapeutically effective amount, which is the amount used to treat the disease. The amount which will be therapeutically effective will depend on the nature of the disorder or condition, and can be determined by standard clinical techniques. In addition, *in vitro* or *in vivo* assays may optionally be employed to help identify optimal dosage ranges. The precise dose to be employed in the formulation will also depend on the route of administration, and the seriousness of the symptoms; and should be decided according to the judgment of a practitioner and each patient's circumstances. Effective doses may be extrapolated from dose-response curves derived from *in vitro* or animal model test systems. In one embodiment of the invention, a therapeutically effective amount for an individual can be an amount which raises the level of adiponectin in a test sample from the individual, so that the level approaches (e.g., is less than two standard deviations below, preferably less than one standard deviation below) or equals a reference level or a control level, as described above.

EXAMPLE 1: Correlation between Adiponectin Levels and Endometrial Cancer *Material and methods*

Eighty-four eligible women with histologically confirmed endometrial cancer were admitted to the First Department of Obstretics and Gynecology (OB) of the University of Athens teaching hospital "Alexandra." All these women were included in this investigation and the vast majority (82%) presented with an early stage I endometrial cancer. For each woman with endometrial cancer, a control woman was enrolled among those admitted during the same week to the same clinical department for small gynecological operations, mainly for pelvic prolapse. Both cases and controls had to be residents of the Greater Athens area and free from any form of current or past malignancy. All study participants provided informed consent and were interviewed in the hospital by the same gynecology resident using the same questionnaire.

Blood samples were collected prior to therapy. A fasting morning blood sample was taken for measurements of adiponectin, major components of the IGF system and leptin. Adiponectin was measured by radioimmunoassay with a sensitivity of 2 ng/mL and the intra-assay coefficient of variation was 8.1 %. IGF-I was run on the Nichols Advantage™ Automated Specially System (Nichols Institute, San Juan Capistrano, CA). No cross-reactivity with IGF-II, Pro-Insulin, Insulin, Thyroid- Stimulating Hormone (TSH) or Luteinising Hormone (LH) was detected. The sensitivity of the assay was 6ng/ml, whereas the intra-assay coefficient of variation was 4.8%. IGF-II was determined by using the DSL-2600 ACTIVE™ Non-Extraction Insulin-Like Growth Factor-II Coated-Tube Immunoradiometric Assay Kit. The procedure employs a two-site immunoradiometric assay (IRMA). The DSL non-extraction IGF-II IRMA kit was used instead of ELISA because the laboratory that run these tests had set up and validated that IRMA assay as more sensitive than the corresponding ELISA assay. The sensitivity was 12ng/ml and the intra assay coefficient of variation was 4.7%. IGFBP-3 concentrations were measured using a commercially available radioimmunoassay kit (IGFBP-3100T kit Nichols Institute, San Juan Capistrano, CA). The sensitivity of the assay was 0.0625 $\mu$g/ml and the intra-assay coefficient of variation 3.8%. Leptin was determined by using DSL-23100 Leptin Coated-Tube Immunoradiometric Assay Kit. The procedure employs a two-site immunoradiometric assay (IRMA) principle designated to detect leptin. The sensitivity of the assay was 0.10 ng/ml and the intra assay coefficient of variation was 2.6%.

For the statistical analysis, representative values of adiponectin were calculated among the apparently healthy control women. Subsequently, serum adiponectin values were evaluated in relation to a series of independent variables in order to identify possible predictors of adiponectin levels among healthy women. In order to study a possible association of adiponectin with endometrial cancer, the data was modeled through multiple logistic regression using case control as outcome variable and adiponectin (in increments of one standard deviation of the compound among controls) and a series of possible confounders as predictor variables. Possible confounders were sociodemographic characteristics, notably age (in 10-year increments), education (in 6-year increments), and established or suspected risk factors for endometrial cancer, specifically height (in 5-cm increments), body mass index (BMI) before onset of symptoms (in 2kg/m$^2$ increments), age at menarche (two categories, with cutoff point at age 14), and parity (two categories: ever or never pregnant). There were too few still menstruating women to allow evaluation of the impact of menopausal status in this investigation. Also hormone replacement therapy was not included as a variable because this practice is uncommon in Greece and is particularly uncommon among low-income women, like those included in the present study. In additional models, IGF-I, IGF-II, IGFBP-3 and leptin were included as covariates, all of them in increments of one standard deviation.

In order to evaluate possible interaction between age and adiponectin in the etiology of endometrial cancer, the analysis was repeated among women younger than 65 years and those 65 or older. The cut off point of 65 years represents the approximate median age in our study sample.

*Results*

The mean value and the standard deviation of adiponectin among healthy women were: 13.53 $\mu$g/mL and 5.26 $\mu$g/mL, respectively. The first, second (median) and third quartiles were: 9.97, 13.21 and 17.68, respectively. The distribution of this hormone deviates little from normality. Table 1 shows the results from the regression of adiponectin on a series of variables that were chosen either for descriptive purposes or because they are known or suspected to be risk factors for endometrial cancer.

Table 1: Multiple regression-derived partial regression coefficients *b* (95% Confidence intervals, CIs) for changes of serum adiponectin levels ($\mu$g/mL) by specified changes of possible predictor variables among 84 healthy women

| Variable | Category or increment | B | 95% | CIs | p-value |
|---|---|---|---|---|---|
| Age | | | | | |

(continued)

| Variable | Category or increment | B | 95% | CIs | p-value |
|---|---|---|---|---|---|
| Education | 10 years | -0.20 | -1.32 | 0.91 | 0.72 |
| | 6 years | -0.48 | -2.30 | 1.34 | 0.61 |
| Height | 5 cm | -0.74 | -1.83 | 0.34 | 0.18 |
| BMI before onset of symptoms | 2 kg/m$^2$ | -0.14 | -0.83 | 0.55 | 0.69 |
| Age at menarche | <14 years | Baseline | | | |
| | 14+ | -1.60 | -3.99 | 0.79 | 0.19 |
| Ever pregnant | No | Baseline | | | |
| | Yes | 5.22 | -1.36 | 11.80 | 0.12 |

There is no evidence that any of the studied variables is an important predictor of serum adiponectin levels among healthy women of relatively advanced age. In particular, there is no evidence for a positive association of adiponectin with BMI, although such an association cannot be excluded on the basis of the confidence interval.

In order to evaluate the association of adiponectin with endometrial cancer, possible confounders were first sought to be identified. Table 2 shows the distribution of women with endometrial cancer and control women by the study variables that were also evaluated in Table 1.

Table 2: Distribution of 84 women with incident endometrial cancer and 84 control women by selected socio-demographic variables and important endometrial cancer risk factors*

| Variable | | Cases | | Controls | | p value for contrast or trend (1 degree of freedom) |
|---|---|---|---|---|---|---|
| | | N | % | N | % | |
| Age | | | | | | 0.67 |
| | <55 years | 17 | 20.2 | 17 | 20.2 | |
| | 55-64 | 27 | 32.1 | 22 | 26.2 | |
| | 65-74 | 31 | 36.9 | 36 | 42.9 | |
| | 75+ | 9 | 10.7 | 9 | 10.7 | |
| Education | | | | | | 0.02 |
| | <6 years | 39 | 46.4 | 27 | 32.2 | |
| | 6-11 | 37 | 44.1 | 40 | 47.6 | |
| | 12+ | 8 | 9.5 | 17 | 20.2 | |
| Height | | | | | | 0.38 |
| | <155 cm | 4 | 4.7 | 12 | 14.3 | |
| | 155-159 | 26 | 31.0 | 16 | 19.0 | |
| | 160-164 | 26 | 31.0 | 31 | 36.9 | |
| | 165-169 | 22 | 26.2 | 22 | 26.2 | |
| | 170+ | 6 | 7.1 | 3 | 3.6 | |
| BMI before onset of symptoms | | | | | | 0.001 |
| | <25 kg/m$^2$ | 17 | 20.2 | 25 | 29.8 | |
| | 25-26 | 13 | 15.5 | 31 | 36.9 | |
| | 27-28 | 18 | 21.4 | 9 | 10.7 | |

(continued)

| Variable | Cases | | Controls | | p value for contrast or trend (1 degree of freedom) |
|---|---|---|---|---|---|
| | N | % | N | % | |
| 29-30 | 13 | 15.5 | 10 | 11.9 | |
| 31+ | 23 | 27.4 | 9 | 10.7 | |
| Age at menarche | | | | | 0.04 |
| <14 years | 65 | 77.4 | 53 | 63.1 | |
| 14+ | 19 | 22.6 | 31 | 36.9 | |
| Ever pregnant | | | | | 0.009 |
| Yes | 71 | 84.5 | 81 | 96.4 | |
| No | 13 | 15.5 | 3 | 3.6 | |

*There were too few premenopausal women or postmenopausal women using hormone replacement therapy

Parity, BMI, age at menarche and educational level could have a confounding influence, whereas age is a variable of central importance and height is a prerequisite for the calculation of BMI. All these variables were controlled for in subsequent models.

It was also evaluated whether other hormones related either to carcinogenesis, such as components of the IGF system, or to adiposity, such as leptin, could confound the association of adiponectin with endometrial cancer. Spearman's correlation coefficients of adiponectin with IGF-I, IGF-II, IGFBP-3 and leptin were, respectively, 0.03, 0.14, 0.05 and -0.01. None of these associations is statistically significant, but to guard against the possibility of joint confounding, it was decided to include these four hormones in some of the models.

In Table 3, the odds ratio for endometrial cancer for an increment of one standard deviation of adiponectin is shown. This odds ratio is derived from various models. Among all women (upper panel of Table 3) the crude odds ratio is 0.83 and is reduced to 0.78 after adjustment for sociodemographic, reproductive and relevant hormonal variables. Nevertheless, the inverse association between adiponectin and endometrial cancer remains statistically non-significant. However, when the association under investigation is separately evaluated among women younger and older than 65 years respectively, *post hoc* evidence for interaction emerges: among younger women, adiponectin is significantly inversely related to endometrial cancer, whereas no such association is noted among older women. The age group - adiponectin interaction with respect to endometrial cancer was statistically significant (p=0.001). It should be noted that in similar multiple logistic regression models, adjusted odds ratios (ORs) and 95% confidence intervals (95% CIs) for endometrial cancer for an increase of adiponectin by *one quintile* (rather than one standard deviation) is statistically significant for the entire study group (0.74, 0.56-0.97) and for the subgroup of women below age 65 (0.51, 0.32-0.81) but not for those 65 years and older (1.03, 0.57- 1.68).

Table 3: Multiple logistic regression-derived, adjusted odds ratios (ORs) and 95% Confidence Intervals (95% CIs) for endometrial cancer for a change in adiponectin by one standard deviation (among controls)

| Variable | ORs | 95% | CIs |
|---|---|---|---|
| *Model 1*: adiponectin only | 0.83 | 0.62 | 1.10 |
| *Model 2*: adiponectin plus covariates in Table 2 | 0.80 | 0.58 | 1.10 |
| *Model 3*: adiponectin plus covariates in Table 2 plus IGF-I, IGF-II, IGF-BP3 and leptin | 0.78 | 0.56 | 1.10 |
| **Women less than 65 years** | | | |
| *Model 1*: adiponectin only | 0.56 | 0.35 | 0.90 |
| *Model 2*: adiponectin plus covariates in Table 2 | 0.50 | 0.30 | 0.85 |

(continued)

| Women less than 65 years | | | |
|---|---|---|---|
| *Model 3*: adiponectin plus covariates in Table 2 plus IGF-I, IGF-II, IGF-BP3 and leptin | 0.44 | 0.24 | 0.81 |
| **Women 65 years or more** | | | |
| *Model 1*: adiponectin only | 1.17 | 0.79 | 1.75 |
| *Model 2*: adiponectin plus covariates in Table 2 | 1.10 | 0.68 | 1.81 |
| *Model 3*: adiponectin plus covariates in Table 2 plus IGF-I, IGF-II, IGF-BP3 and leptin | 1.26 | 0.73 | 2.18 |

*Discussion*

The results of the present case-control study suggest an inverse association of serum adiponectin levels with endometrial carcinoma. The observed association is highly significant in younger women (age < 65 years) but is not present in older women. The inverse association noted in the younger age group was strengthened after adjustment for potential confounders such as age, BMI, known reproductive risk factors for EC, hormones that have been linked to carcinogenesis (IGF-1, IGF-2, and IGFBP-3), and leptin, an hormone associated with body fat mass.

Obesity is a known risk factor for endometrial cancer, with the purported mechanism being increased peripheral aromatization of adrenal androgens to estrogens in adipose tissue leading to increased circulating estrogens. While endometrial cancer is primarily a disease of post-menopausal women, a fraction of cases are found in pre-menopausal women (Gallup, D.G. and Stock, R.J., Obstet Gynecol 1984; 64:417-20; Peterson, E.P., Obstet Gynecol 1968; 31:702-7.). Epidemiologic studies have shown that of women diagnosed with EC, obesity is more prevalent in premenopausal compared to postmenopausal women (Gallup, D.G. and Stock, R.J., Obstet Gynecol 1984; Evans-Metcalf, E.R., et al.,. Obstet Gynecol 1998; 91: 349-354), although it is important to note that these studies may have included premenopausal women with polycystic ovarian syndrome, a disease characterized by chronic anovulation, increased circulating androgens, insulin resistance, and obesity. In the present study, there were too few menstruating women enrolled to meaningfully compare pre-menopausal and post-menopausal women with EC. One could speculate that the significant inverse association of adiponectin with Endometrial cancer in the younger women in our study may be related with an increased prevalence of obesity in this subgroup.

Insulin resistance, characterized by hyperinsulinemia and frequently co-existing with obesity, has been associated with Endometrial cancer (Rutanen, E.M. et al., J Clin Endocrinol Metab 1993; 77:199-204; Nagamani, M. et al., J Clin Endocrinol Metab 1988; 67:144-148). Type 2 diabetes, a disease state characterized by early hyperinsulinemia and persistent insulin resistance has also been linked to Endometrial cancer (Brinton, L.A. et al., Am J Obstet Gynecol 1992; 167:1317-1325; Weiderpass, E. et al., Cancer Causes Control 2000; 11:185-192; La Vecchia, C. et al., Br J Cancer 1994; 70:950-953.). Insulin was initially hypothesized to be a mitogen because it induces mammary carcinomas in rodents (Lupulescu, A.P., Cancer Res 1985; 45:3288-95; Hueson, J.C. and Legro, N., Cancer Res 1972; 31:226-32). It is now believed that insulin stimulates the growth of endometrial stromal cells through direct binding to insulin receptors (IR) on endometrial cell membranes (Nagamani, M. and Stuart, C.A., Am J Obstet Gynecol 1998; 179(1):6-12). Other studies have shown that insulin-like growth factors also bind to IGF receptors found on endometrial cell membranes and, along with insulin, may potentiate endometrial carcinogenesis (Sheets, E.E. et al., Am J Obstet Gynecol 1985; 153: 60-5; Nagamani, M. et al., Am J Obstet Gynecol 1991: 165:1865-71; Surrey, E. et al., abstract 506 in: Proceedings of the Thirty-eighth Annual Meeting of the Society for Gynecologic Investigation; 20-23 Mar 1991; San Antonio, Texas. San Antonio: The Society; 1991). Because insulin and insulin-like growth factors can bind to both the respective receptors, a role of hyperinsulinemia in the pathogenesis of Endometrial cancer can be inferred.

Additionally, recent *in vitro* studies have demonstrated that insulin up-regulates the secretion and mRNA expression of vascular endothelial growth factor, a potent angiogenic factor that may contribute to an increased risk for Endometrial cancer (Bermont, L. et al., J Clin Endocrinol Metab 2001; 86(1):363-8; Mick, G.J. et al., Endocrinology 2002; 143(3):948-53.).

Little is known about the regulation of adiponectin secretion or its mechanism of action. Prior studies have demonstrated an inverse association of adiponectin with obesity, type 2 diabetes mellitus, insulin resistance, and congenital lipodystrophic syndromes (Hu, E. et al., J Biol Chem 1996; 271:10697-10703; Arita, Y. et al., Biochem Biophy Res Commun 1999; 257:79-83; Weyer, C. et al., J Clin Endocrinol Metab 2001; 86:1930-1935; Hotta, K. et al., Arterioscler Thromb Vasc Biol 2000; 20:1595-1599; Haque, W.A. et al., J. Clin. Endocrinol. Metab. 2002; 87(5):2395-98) Visceral fat is linked to metabolic abnormalities such as insulin resistance (Peiris, A.N. et al., Acta Med. Scand. Suppl. 1999; 723:179-188; Fujioka, S. et al., Int. J. Obes. 1990; 15:853-859). Based upon *in vivo* animal studies where adiponectin reduced insulin resistance when administered to lipodystrophic mice with diabetes mellitus and hypoadiponectinemia, adiponectin appears to act as an insulin sensitizer (Yamauchi, T. et al., Nature Med. 2001; 7:941-946).

In the present study, reduced adiponectin levels in younger women may reflect increased insulin resistance, which is associated with Endometrial cancer possibly through an interaction with circulating estrogens that potentiate the effect of low adiponectin levels by sensitising the endometrium to circulating insulin and one or more of the insuline like growth factors. In conclusion, evidence was found that among women younger than 65 years adiponectin is inversely related to the risk of Endometrial cancer and this association is independent of possible effects of IGF-1, IGF-2, IGF-BP3, leptin and gynaecological risk factors of the disease.

EXAMPLE 2 Confirmation of Correlation between Adiponectin Level and Endometrial Cancer

A case-control study of endometrial cancer was conducted between 1999 and 2002 in Pordenone (North-Eastern Italy). Cases were 87 women, aged 34-78 years (median age 62) with incident, histologically confirmed endometrial cancer. Three (4%) cases had stage 0, 50 (62%) had stage I,11 (14%) stage II, and 17 (21%) stage III or IV. Controls were 132 women, aged 29-79 years (median age 61) who had an intact uterus and had been admitted to the same hospital network for acute non-neoplastic conditions unrelated to gynecologic, hormonal, or metabolic disorders or to dietary modifications. Thirty-two percent of controls were admitted for traumas, 55% for non-traumatic orthopedic diseases, and 13% for other miscellaneous illnesses such as eye, nose, throat or dental disorders.

Information was collected by trained interviewers in hospital wards on sociodemographic and anthropometric characteristics, smoking habits, physical activity, height and weight, selected medical conditions, menstrual and reproductive factors, and use of hormone replacement therapy (HRT). To assess the diet, including total energy intake, a validated food frequency questionnaire was used including 78 foods, food groups, or recipes (Franceschi, S. et al., Ann Epidemiol. 1995;5:69-75).

No cases and two controls (1.4%) refused the interview. All study participants provided a written informed consent. Blood samples were drawn before cancer therapy at the time of interview. They were immediately centrifuged and stored with EDTA at -80C until shipment in dry ice to the Human Nutritional Research Unit, Boston, United States for testing. Adiponectin analysis was performed, by means of a radioimmunoassay (RIA) than as a sensitivity of 2ng/ml, and intraassay coefficient of variation of 8% (Petridou, E. et al., J. Clin. Endocrinol Metab. 2003;88:993-7).

OR, and the corresponding 95% confidence intervals (CI), for tertiles of plasma and serum adiponectin, were computed using unconditional multiple logistic regression models, including terms for age and BMI (kg/m$^2$), education, parity, smoking status, and history of diabetes and HRT.

*Results*

Cases were more frequently overweight (OR=5.87 for BMI≥30 *vs.* BMI<25), used less frequently oral contraceptives (OR=0.75 for ever *vs.* never users), and had a higher intake of total energy than control women (OR=2.12 for the highest tertile of energy intake *vs.* the lowest). Only 11 % of cases and 17% of controls had ever used HRT, generally for less than 2 years (OR=0.43). Plasma adiponectin were weakly correlated with age (Spearman r=0.09), energy intake (r= -0.11), and BMI (r=-0.24).

Table 4 shows the associations of endometrial cancer with plasma and serum adiponectin levels. The OR was 0.42 (95% CI: 0.19-0.94) for the highest tertile of plasma adiponectin and 0.30 (95% CI: 0.14-0.68) for the highest tertile of serum adiponectin. Premenopausal women showed a stronger inverse association with levels of plasma adiponectin (OR=0.06; 95% CI: 0.00-0.73).

The combined effect of plasma adiponectin and BMI in endometrial cancer risk in women is shown in Table 5. Compared to low BMI and high plasma adiponectin, the OR for high BMI and low plasma adiponectin was 6.45 (95% CI: 2.55-16.35). Similar results were seen for serum adiponectin (OR=10.17, 95% CI: 3.82-27.09). Additional analyses with respect to the combination of high total energy intake (≥2300 Kcal) and low adiponectin levels led to an OR of 2.75 (95% CI: 1.16-6.52) for plasma and 3.19 (95% CI: 1.36-7.44) for serum adiponectin. The exclusion of patients diagnosed at stage III or IV (17 cases) did not materially change any of the results. Moreover, the additional adjustment for waist-to-hip ratio (WHR) did not appreciably modify the results significantly and no effect modification was seen by WHR.

*Discussion*

The present analysis provides additional strong evidence that serum and plasma levels of adiponectin are inversely and independently related to endometrial cancer risk, even after allowance for BMI and other major identified potential confounding factors (Yannakoulia, M. et al. J. clin. Endocrinol. Metab., 2003;88:1780-6). Consequently, the combination of high BMI and low adiponectin levels led to over 6-fold excess risk. As in Greek study described in Example 1, the inverse association was apparently stronger in younger women, and particularly in pre-menopausal women. The inclusion of young women with anovulation or polycystic ovary syndrome (PCOS) (Evans-Metcalf, E.R. et al., Obstet Gynecol. 1998;91:349-54; Parazzini, F. et al., Gynecol Oncol 1991;41:1-16), a disease characterised by several factors directly associated with endometrial cancer (Parazzini, F. et al., Gynecol Oncol 1991;41:1-16; Kaaks, R. et al., Cancer Epidemiol Biomarkers Prev 2002;11:1531-43; Kauffman, R.P. et al., Am J Obstet Gynecol 2002;187:1362-9), may play a role.

The observation that overweight and adiponectin have independent roles in endometrial cancer risk indicates that the two mechanisms - excess estrogen levels and insulin resistance - may act independently in endometrial carcinogenesis. It was also observed that a diet with high glycemic index and load - which are related to high levels of blood glucose, insulin and possibly insulin-like growth factors - are directly related to endometrial cancer risk (Augustin, L.S. et al., Int J Cancer 2003;105:404-7).

Although the study was hospital- based, it is unlikely that bias or confounding substantially influenced its main findings, since the catchment areas of cases and controls were similar, participation was practically complete, major identified risk factors were consistent with our knowledge of endometrial carcinogenesis, and allowance was possible for major potential confounding factors. Data collection for all cases and controls was made before any treatment, and it is therefore unlikely that the development of endometrial cancer or any other disease may have affected adiponectin measures. Interview and blood collection was made in the majority of study women on the first day of hospital admission and, for cancer cases, always before they had undergone surgical or radiation treatment. Furthermore, analyses of adiponectin in serum and plasma samples yielded consistent results.

**Table 4**. Odds ratios (OR)[*] and corresponding 95% confidence intervals (CI) of endometrial cancer according to plasma and serum levels of adiponectin ($\mu$g/ml) in the total population and in different strata of menopausal status.

| | Cases[†] | Controls[†] | OR (95% CI) | $\chi_1^2$ trend (p-value) |
|---|---|---|---|---|
| **Adiponectin, plasma** | | | | |
| <10 | 38 | 35 | 1 | |
| 10-18 | 24 | 42 | 0.51 (0.24-1.08) | |
| >=19 | 19 | 50 | 0.42 (0.19-0.94) | 4.68 (p=0.03) |
| **Menopausal status** | | | | |
| Pre-peri menopausal | | | | |
| <10 | 9 | 10 | 1 | |
| 10-18 | 7 | 10 | 0.11 (0.01-1.28) | |
| >=19 | 3 | 11 | 0.06 (0.00-0.73) | 5.21 (p=0.42) |
| Postmenopausal | | | | |
| <10 | 29 | 25 | 1 | |
| 10-18 | 17 | 32 | 0.59 (0.25-1.41) | |
| >=19 | 16 | 39 | 0.58 (0.23-1.44) | 1.47 (p=0.23) |

(continued)

| Adiponectin, serum | | | | |
|---|---|---|---|---|
| <13 | 40 | 36 | 1 | |
| 13-23 | 30 | 42 | 0.55 (0.27-1.14) | |
| >=24 | 17 | 54 | 0.30 (0.14-0.68) | 8.50 (p<0.01) |
| **Menopausal status** | | | | |
| Pre-peri menopausal | | | | |
| <13 | 12 | 15 | 1 | |
| 13-23 | 5 | 6 | 0.51 (0.08-3.46) | |
| >=24 | 3 | 10 | 0.21 (0.03-1.28) | 2.91 (p=0.09) |
| Postmenopausal | | | | |
| <13 | 28 | 21 | 1 | |
| 13-23 | 25 | 36 | 0.50 (0.22-1.15) | |
| >=24 | 14 | 44 | 0.25 (0.10-0.64) | 8.45 (p<0.01) |

[*] Estimates from multiple logistic regression equations, including terms for age, education, parity, smoking status, body mass index, and hormone replacement therapy.
[†] The sum does not add up to the total because of some missing values.

**Table 5.** Odds ratios (OR)[*] and corresponding 95% confidence intervals (CI) of endometrial cancer according to the combined effect of plasma levels of adiponectin ($\mu$g/ml) and body mass index (BMI, kg/m$^2$).

| | BMI | | | |
|---|---|---|---|---|
| | <26 | | ≥26 | |
| | Case:controls | OR (95%CI) | Case:controls | OR (95%CI) |
| **Plasma adiponectin** | | | | |
| ≥19 | 11:40 | 1[†] | 14:15 | 3.37 (1.14-9.36) |
| 10-18 | 8:21 | 1.38 (0.47-4.05) | 16:21 | 2.49 (0.95-6.51) |
| <10 | 9:18 | 1.81 (0.61-5.36) | 29:17 | 6.45 (2.55-16.35) |

[*] Estimates from multiple logistic regression equations, including terms for age, education, parity, smoking status, BMI, and hormone replacement therapy.
[†] Reference category

EXAMPLE 3 Correlation between Adiponectin and Breast Cancer

*Materials and Methods*

*Subjects*

During an 8-month period from February to September 1998 inclusive, 83 consecutive incident cases of breast cancer were diagnosed and histologically confirmed in the mammographic screening centres of the University of Athens teaching hospitals "E. Venizelou" and "Laiko". Five of these women refused to participate, whereas three others had a past history of cancer at another site. The remaining 75 cases were included in the study. Controls were selected among women with a mammogram indicating the absence of breast cancer and who had never been diagnosed with any type of cancer. Of 97 identified potential controls, 86 agreed to participate. During an additional 30- month period, from January 2000 to June 2002 inclusive, visited the mammographic screening centres of the above teaching hospitals were visited once a week to identify potential cases. Cases included women who were histologically diagnosed with breast cancer during the present hospitalisation. Among the 118 women who were identified, 99 agreed to participate and were included in the study. Controls were selected among women in the same hospitals who either had a mammogram indicating the absence of breast cancer or who were hospitalised in the orthopaedic department for a minor trauma. Controls were included if they had never been diagnosed with any form of cancer. Among the 118 potential controls that were identified, 92 agreed to participate and were included in the study.

All cases and controls were interviewed by one of four trained interviewers. The interview lasted about 20 minutes and obtained information pertaining to demographic, anthropometric, and reproductive variables. Fasting blood samples were taken and stored at -70° C from all cases and controls (no later than 9 a.m.) in a blinded fashion as to case control status for measurements of serum adiponectin, leptin, IGF-I, and IGF binding protein 3 (IGFBP-3).

*Ethics*

The study protocol was approved by the University of Athens Medical School Ethical Committee, and was in accordance with the Helsinki Declaration of 1975. All participants provided informed consent.

*Hormone Measurement*

Serum adiponectin levels in all samples were measured in one run at the Beth Israel Deaconess Medical Centre (Boston, MA USA) by radioimmunoassay with a sensitivity of 2 ng/mL and an intra-assay coefficient of variation of 8.1%. Measurements of serum IGF-I, IGFBP-3, and leptin were performed in two runs (set A and set B: each including a similar number of cases and controls) using either the Nichols Advantage™ Automated Specialty System (Nichols Institute, San Juan Capistrano, CA) or commercially available radioimmunoassay kits as previously described. (20-21). The assays for these analytes are similar with respect to sensitivity, specificity, precision, recovery and linearity of dilution; thus, the methods are considered as generating comparable results.

*Statistical analysis*

Because leptin and components of the IGF system were analysed in two different runs, a dummy variable specifying the contrast between set A and set B was introduced in all analyses, even though the laboratory methods used were similar and cases and controls were distributed in a balanced way between the two runs. Additionally, even though all samples were immediately frozen after blood collection and processing, it is theoretically possible that the duration of storage might have affected measurements of the four indicated hormones. Thus, for each hormone, a regression of hormonal measurements on duration of storage was obtained and residuals (differences) from the regression-predicted values were used in all subsequent analyses as storage duration-adjusted values.

For the statistical analysis, representative values (mean, standard deviation) of the four measured hormones were calculated among the case and control subjects and were stratified according to menopausal status. Subsequently, cases and controls were distributed in marginal quintiles of the storage duration-adjusted values for each of the hormonal variables, and p-values from simple test trends were determined. Lastly, the data were modelled through multiple logistic regression with case or control status as the outcome variable and one or more of the measured hormones as predictor variables (in increments equal to one marginal quintile of their storage duration-adjusted values). Models were controlled for age, education, height, body mass index (BMI), age at menarche, alcohol consumption, tobacco use, age at menopause (among postmenopausal women), and age at first birth (among parous women), as well as for inclusion in set A or set B.

*Results*

Table 6 shows the distribution of 174 women with incident breast cancer and 167 control women by demographic, anthropometric, and reproductive variables. These data are not directly interpretable because of mutual confounding. However, they reveal most of the established risk characteristics of women with breast cancer, including higher level of education (p = 0.05) and increased stature (p = 0.001); earlier age at menarche (p = 0.001); later age at menopause (p = 0.004); and their tendency to consume more alcoholic beverages (p = 0.001). BMI tended to be higher in cases compared to controls, however this difference did not achieve statistical significance (p = 0.25).

Table 7 shows mean values and standard deviations of the measured hormones among women with breast cancer and control women by menopausal status. No significant differences between cases and controls are noted with respect to any of the hormones, especially given the multiple comparisons performed herein. However, the values in Table 7 are not adjusted for either inclusion in set A or B or for storage duration. Therefore, Table 7 serves only rough descriptive purposes.

Table 8 shows the distribution of women with breast cancer and control women by marginal quintiles of storage duration-adjusted measurements of the four indicated hormones according to menopausal status. Adiponectin is inversely associated with breast cancer risk among postmenopausal women (p = 0.02), and this association is also reflected among all women (p = 0.02), probably because most women with breast cancer in our study were postmenopausal (71.8% of cases).

Table 9 shows multiple logistic regression-derived odds ratios (ORs) and 95% confidence intervals (CIs) for breast cancer according to a change in serum adiponectin, IGF-I, IGFBP-3, and leptin by one marginal quintile of the storage duration-adjusted measurements stratified by menopausal status. For IGF-I, there tends to be a positive association with breast cancer risk among premenopausal women (p = 0.45), which becomes more significant after controlling for the other measured hormones (p = 0.06). For IGFBP-3, there is an inverse association with breast cancer risk among premenopausal women (p=0.13), which also becomes more significant after controlling for the other measured hormones (p = 0.01). An inverse association of serum leptin levels and risk of breast cancer (p = 0.32) does not achieve statistical significance among premenopausal women in unadjusted analysis or after controlling for the other measured hormones (p = 0.12). There is no evidence for an association of IGF-I, IGFBP-3 and leptin with breast cancer risk among postmenopausal women; however, there is evidence for a fairly robust inverse association of adiponectin with breast cancer risk among postmenopausal women (OR = 0.82,95% CI 0.67-1.00), which is also observed in the entire data set (OR = 0.84, 95% CI 0.71- 0.99). In contrast, there is no evidence for a significant inverse association between adiponectin and breast cancer risk among premenopausal women.

*Discussion*

The results of this case-control study demonstrate an inverse association of adiponectin with the risk of post-menopausal, but not pre-menopausal, breast cancer. As in previous studies (Hankinson, S. et al., 1998 Lancet 351: 1393-6), there is evidence in these data that IGF-I is positively and IGFBP-3 inversely associated with the risk for the development of pre-menopausal but not postmenopausal breast cancer. The apparent differences in the associations between these hormonal factors and the risk for the development of breast cancer in pre- and postmenopausal periods may be due to important differences in the pathogenesis of these disease states and need to be studied further.

Previous epidemiologic studies have shown an association of central obesity and insulin resistance mainly with postmenopausal breast cancer (Stoll, B.A., 2002 Int J Obes Relat Metab Disord 26:747-53; Michels, K.B. et al., 2003 Diabetes Care 26:1752-8; Stoll, B.A.1999, Eur J. Clin. Nutr 54:83-7). Similarly, overall obesity, expressed as BMI, tends to be positively correlated with the risk of postmenopausal breast cancer but is either weakly or inversely associated with pre-menopausal breast cancer (Cleary, M.P. and Maihle, N.J., 1997, Proc. Soc. Exp. Biol. Med 216: 28-42; Franceschi, S. et al., 1996 Int. J. Cancer 67:181-6; Franceschi, S. et al., 1996 Int J. Cancer 67:181-6; van den Brandt, P.A., et al. 2000 Am J Epidemiol. 152:514-27). These observations suggest that central obesity and insulin resistance, characterized by increased serum insulin levels, may play a more important role in the pathogenesis of postmenopausal breast cancer.

Adiponectin is secreted exclusively by adipoctyes and acts as an insulin sensitiser. Finding a similar inverse association among postmenopausal women with breast cancer in this study, as above in relation to endometrial cancer, provides further support to the importance of adiponectin in the pathogenesis of malignancies associated with obesity-induced insulin resistance and hyperinsulinemia. These studies suggest that low levels of adiponectin may play a permissive role in stimulating the neoplastic growth of breast cells.

In contrast to the role of adiponectin observed in postmenopausal women, there was not an association of adiponectin with pre-menopausal breast cancer. There was, however, a positive association of IGF-I, and an inverse association of IGFBP3, with the risk for development of pre-menopausal breast cancer.

Pathophysiologically, IGF-I appears to increase mitogenic stimulation of breast cells through both endocrine and paracrine mechanisms, and its effects may synergise with the mitogenic effects of oestrogen. That IGF-I is positively correlated with the risk for pre-menopausal but not postmenopausal breast cancer may imply the importance of this hormone in the earlier stages of carcinogenesis and in subjects who have higher endogenous levels of both IGF-I and estrogens.

Among the strengths of this study are the inclusion of newly diagnosed pre-and postmenopausal women with a histological diagnosis of breast cancer. Laboratory specimens were obtained in a blinded fashion, and specimens were obtained in the fasting state to minimize diurnal variability in hormone levels. Random laboratory error or uncontrolled variability would have resulted in misclassification that would tend to dilute associations. Although subjects were recruited from two different sites and laboratory analyses were performed in two different runs, we made proper adjustments for these conditions in the statistical analyses. Lastly, the potential variability in hormonal levels due to storage duration time was taken into consideration.

In conclusion, a significant inverse association of adiponectin with postmenopausal breast cancer and a positive association of IGF-I with pre-menopausal breast cancer were found. These observations support important underlying pathophysiologic differences in these two disease states.

Table 6: Distribution of 174 women with breast cancer and 167 control women by demographic, somatometric and reproductive variables

| Variables | | Cases | | Controls | | p-value for trend or contrast |
|---|---|---|---|---|---|---|
| | | N | % | N | % | |
| Age | | | | | | 0.62 |
| | <45 years | 24 | 13.8 | 25 | 15.0 | |
| | 45-54 | 38 | 21.8 | 33 | 19.7 | |
| | 55-64 | 40 | 23.0 | 32 | 19.2 | |
| | 65-74 | 52 | 29.9 | 54 | 32.3 | |
| | 75+ | 20 | 11.5 | 23 | 13.8 | |
| Education | | | | | | 0.05 |
| | <6 years | 23 | 13.2 | 39 | 23.3 | |
| | 6 | 51 | 29.3 | 58 | 34.7 | |
| | 9 | 46 | 26.5 | 22 | 13.2 | |
| | 12 | 32 | 18.4 | 26 | 15.6 | |
| | 13+ | 22 | 12.6 | 22 | 13.2 | |
| Alcohol consumption (glasses) | | | | | | 0.001 |
| | <1/week | 120 | 69.0 | 146 | 87.4 | |
| | ≥ 1/week | 54 | 31.0 | 21 | 12.6 | |
| Smoking | | | | | | 0.54 |
| | no | 124 | 71.3 | 124 | 74.3 | |
| | yes /ex-smoker | 50 | 28.7 | 43 | 25.7 | |
| Height | | | | | | 0.001 |
| | <160 cm | 37 | 21.3 | 46 | 27.5 | |
| | 160-164 | 54 | 31.0 | 82 | 49.1 | |
| | 165+ | 83 | 47.7 | 39 | 23.4 | |
| Body mass index | | | | | | 0.25 |
| | <25.0 kg/m$^2$ | 69 | 39.7 | 77 | 46.1 | |
| | 25.0-26.9 | 33 | 19.0 | 30 | 18.0 | |
| | 27.0-28.9 | 31 | 17.8 | 26 | 15.6 | |
| | 29.0+ | 41 | 23.5 | 34 | 20.3 | |
| Age at menarche | | | | | | 0.001 |
| | <13 years | 66 | 38.0 | 36 | 21.6 | |
| | 13 | 56 | 32.2 | 49 | 29.3 | |
| | 14 | 26 | 14.9 | 50 | 29.9 | |
| | 15+ | 26 | 14.9 | 32 | 19.2 | |
| Age at menopause | | | | | | 0.004 |
| | premenopausal | 49 | 28.2 | 44 | 26.4 | |
| | ≤49 years | 41 | 23.5 | 66 | 39.5 | |
| | 50+ | 84 | 48.3 | 57 | 34.1 | |
| Age at first birth | | | | | | 0.36 |
| | nulliparous | 26 | 14.9 | 27 | 16.2 | |
| | <30 years | 107 | 61.5 | 111 | 66.5 | |
| | 30+ | 41 | 23.6 | 29 | 17.3 | |

Table 7: Basic characteristics (mean, standard deviation -SD, and p-value) from comparison of the means for adiponectin, IGF-I, IGFBP-3 and leptin among 174 women with breast cancer and 167 control women by menopausal status.

| Variable | All women (174 cases, 167 controls) | | | Premenopausal women ( 49 cases, 44 controls) | | | Postmenopausal women (125 cases, 123 controls) | | |
|---|---|---|---|---|---|---|---|---|---|
| | mean | SD | p-value (t-test) | mean | SD | p-value (t-test) | mean | SD | p-value (t-test) |
| Adiponectin (μg/mL) | | | 0.54 | | | 0.35 | | | 0.31 |
| cases | 16.7 | 10.0 | | 14.5 | 7.8 | | 17.6 | 10.6 | |
| controls | 17.4 | 10.5 | | 13.0 | 7.1 | | 19.0 | 11.1 | |
| IGF-I (ng/mL) | | | 0.13 | | | 0.83 | | | 0.04 |
| cases | 130.7 | 83.4 | | 175.0 | 94.6 | | 113.0 | 71.4 | |
| controls | 145.2 | 91.1 | | 179.6 | 113.5 | | 133.2 | 78.8 | |
| IGFBP-3 (μg/mL) | | | 0.32 | | | 0.64 | | | 0.42 |
| cases | 3.40 | 1.28 | | 3.81 | 1.27 | | 3.24 | 1.25 | |
| controls | 3.27 | 1.19 | | 3.70 | 1.17 | | 3.11 | 1.16 | |
| Leptin (μg/mL) | | | 0.88 | | | 0.23 | | | 0.44 |
| cases | 24.4 | 16.1 | | 18.7 | 12.5 | | 26.6 | 16.9 | |
| controls | 24.1 | 18.4 | | 22.0 | 14.5 | | 24.9 | 19.6 | |

Table 8: Distribution of women with breast cancer and control women by marginal quintiles of storage duration adjusted measurement of the four indicated hormones by menopausal status

| | 1st | | 2nd | | 3rd | | 4th | | 5th | | trend (+/-), p-value |
|---|---|---|---|---|---|---|---|---|---|---|---|
| | N | % | N | % | N | % | N | % | N | % | |
| Variable | All women: 174 cases, 167 controls | | | | | | | | | | |
| Adiponectin | | | | | | | | | | | (-) 0.02 |
| cases | 35 | 20.1 | 43 | 24.7 | 35 | 20.1 | 31 | 17.8 | 30 | 17.3 | |
| controls | 30 | 18.0 | 24 | 14.4 | 31 | 18.6 | 40 | 23.9 | 42 | 25.1 | |
| IGF-I | | | | | | | | | | | (-) 0.56 |
| cases | 31 | 18.1 | 47 | 27.5 | 25 | 14.6 | 35 | 20.5 | 33 | 19.3 | |
| controls | 37 | 22.3 | 20 | 12.0 | 42 | 25.3 | 33 | 19.9 | 34 | 20.5 | |
| IGFBP-3 | | | | | | | | | | | (-) 0.34 |
| cases | 38 | 21.8 | 33 | 19.0 | 39 | 22.4 | 32 | 18.4 | 32 | 18.4 | |
| controls | 31 | 18.5 | 35 | 21.0 | 28 | 16.8 | 37 | 22.2 | 36 | 21.5 | |
| Leptin | | | | | | | | | | | (+) 0.29 |
| cases | 33 | 19.0 | 38 | 21.8 | 29 | 16.7 | 38 | 21.8 | 36 | 20.7 | |
| controls | 32 | 19.2 | 40 | 23.9 | 39 | 23.3 | 29 | 17.4 | 27 | 16.2 | |

(continued)

| Premenopausal women: 49 cases, 44 controls | | | | | | | | | | |
|---|---|---|---|---|---|---|---|---|---|---|
| Adiponectin | | | | | | | | | | (-) 0.60 |
| cases | 10 | 20.4 | 15 | 30.6 | 11 | 22.5 | 10 | 20.4 | 3 | 6.1 |
| controls | 12 | 27.3 | 6 | 13.6 | 11 | 25.0 | 11 | 25.0 | 4 | 9.1 |
| IGF-I | | | | | | | | | | (+) 0.45 |
| cases | 5 | 10.2 | 6 | 12.2 | 7 | 14.3 | 11 | 22.5 | 20 | 40.8 |
| controls | 7 | 16.3 | 5 | 11.6 | 7 | 16.3 | 8 | 18.6 | 16 | 37.2 |
| IGFBP-3 | | | | | | | | | | (-) 0.13 |
| cases | 8 | 16.3 | 8 | 16.3 | 13 | 26.6 | 8 | 16.3 | 12 | 24.5 |
| controls | 4 | 9.1 | 5 | 11.4 | 10 | 22.7 | 11 | 25.0 | 14 | 31.8 |
| Leptin | | | | | | | | | | (-) 0.32 |
| cases | 14 | 28.6 | 15 | 30.6 | 5 | 10.2 | 10 | 20.4 | 5 | 10.2 |
| controls | 6 | 13.6 | 13 | 29.6 | 13 | 29.5 | 8 | 18.2 | 4 | 9.1 |
| Postmenopausal women: 125 cases, 123 controls | | | | | | | | | | |
| Adiponectin | | | | | | | | | | (-) 0.02 |
| cases | 25 | 20.0 | 28 | 22.4 | 24 | 19.2 | 21 | 16.8 | 27 | 21.6 |
| controls | 18 | 14.6 | 18 | 14.6 | 20 | 16.3 | 29 | 23.6 | 38 | 30.9 |
| IGF-I | | | | | | | | | | (-) 0.16 |
| cases | 26 | 21.3 | 41 | 33.6 | 18 | 14.7 | 24 | 19.7 | 13 | 10.7 |
| controls | 30 | 24.4 | 15 | 12.2 | 35 | 28.5 | 25 | 20.3 | 18 | 14.6 |
| IGFBP-3 | | | | | | | | | | (-) 0.76 |
| cases | 30 | 24.0 | 25 | 20.0 | 26 | 20.8 | 24 | 19.2 | 20 | 16.0 |
| controls | 27 | 22.0 | 30 | 24.4 | 18 | 14.6 | 26 | 21.1 | 22 | 17.9 |
| Leptin | | | | | | | | | | (+) 0.07 |
| cases | 19 | 15.2 | 23 | 18.4 | 24 | 19.2 | 28 | 22.4 | 31 | 24.8 |
| controls | 26 | 21.1 | 27 | 22.0 | 26 | 21.1 | 21 | 17.1 | 23 | 18.7 |

Table 9: Multiple logistic regression -derived odds ratios (ORs) and 95% Confidence Intervals (95% CIs) for breast cancer for a change in serum adiponectin, IGF-I, IGFBP-3 and leptin by one marginal quintile of the storage duration adjusted measurements by menopausal status

| Variable | all women | | | premenopausal | | | postmenopausal | | |
|---|---|---|---|---|---|---|---|---|---|
| | ORs | 95% | CIs | ORs | 95% | CIs | ORs | 95% | CIs |
| Model 1: adiponectin only | 0.83 | 0.72 | 0.97 | 0.92 | 0.66 | 1.27 | 0.81 | 0.68 | 0.96 |
| Model 2: adiponectin plus covariates in table 1 | 0.85 | 0.72 | 1.00 | 0.87 | 0.60 | 1.26 | 0.83 | 0.68 | 1.00 |

(continued)

| Variable | all women | | | premenopausal | | | postmenopausal | | |
|---|---|---|---|---|---|---|---|---|---|
| | ORs | 95% | CIs | ORs | 95% | CIs | ORs | 95% | CIs |
| Model 3: adiponectin plus covariates in table 1 plus IGF-I, IGFBP-3, leptin plus set A vs. B | 0.84 | 0.71 | 0.99 | 0.81 | 0.55 | 1.20 | 0.82 | 0.67 | 1.00 |
| Model 1: IGF-I only | 0.96 | 0.82 | 1.11 | 1.11 | 0.84 | 1.49 | 0.87 | 0.72 | 1.06 |
| Model 2: IGF-I plus Covariates of table 1 | 1.00 | 0.84 | 1.19 | 1.17 | 0.84 | 1.62 | 0.95 | 0.76 | 1.19 |
| Model 3: IGF-I plus covariates of table1 plus adiponectin, IGFBP-3, leptin plus set A vs. B | 1.06 | 0.86 | 1.30 | 1.49 | 0.98 | 2.24 | 0.94 | 0.72 | 1.23 |
| Model 1: IGFBP-3 only | 0.93 | 0.80 | 1.08 | 0.79 | 0.58 | 1.07 | 0.97 | 0.82 | 1.16 |
| Model 2: IGFBP-3 plus Covariates of table 1 | 0.92 | 0.78 | 1.09 | 0.78 | 0.55 | 1.09 | 0.98 | 0.80 | 1.22 |
| Model 3: IGFBP-3 plus ovariates in table 1 plus adiponectin, IGF-I leptin plus set A vs. B | 0.89 | 0.73 | 1.09 | 0.60 | 0.39 | 0.92 | 1.03 | 0.80 | 1.33 |
| Model 1: leptin only | 1.08 | 0.93 | 1.27 | 0.85 | 0.62 | 1.17 | 1.18 | 0.99 | 1.41 |

(continued)

| Variable | all women | | | premenopausal | | | postmenopausal | | |
|---|---|---|---|---|---|---|---|---|---|
| | ORs | 95% | CIs | ORs | 95% | CIs | ORs | 95% | CIs |
| Model 2: leptin plus Covariates of table 1 | 1.00 | 0.83 | 1.21 | 0.77 | 0.52 | 1.14 | 1.05 | 0.84 | 1.31 |
| Model 3: leptin plus covariates in table 1 plus adiponectin, IGF-I, IGFBP-3 plus set A vs. B | 0.97 | 0.80 | 1.18 | 0.72 | 0.47 | 1.10 | 1.00 | 0.80 | 1.27 |

Those skilled in the art will know, or be able to ascertain, using no more than routine experimentation, many equivalents to the specific embodiments of the invention described herein. These and all other equivalents are indented to be encompassed by the following claims.

## Claims

1. An in-vitro method comprising assessing a test sample from a woman who is under 65 years of age for the level of adiponectin, wherein: i) the presence of a level of adiponectin that is equal to or less than a reference level is indicative of the presence of endometrial cancer; ii) the presence of a level of adiponectin that is greater than a reference level is indicative of the absence of endometrial cancer; iii) the presence of a level of adiponectin that is less than a control level, by an amount that is statistically significant, is indicative of the presence of endometrial cancer; iv) the presence of a level of adiponectin that is greater than a control level, by an amount that is statistically significant, or is equal to a control level, is indicative of the absence of endometrial cancer; v) the presence of a level of adiponectin that is less than a level of adiponectin in a comparable negative control sample, by an amount that is statistically significant, is indicative of the presence of endometrial cancer; or vi) the presence of a level of adiponectin that is greater than a level of adiponectin in a comparable negative control sample, by an amount that is statistically significant, or is equal to a level of adiponectin in a comparable negative control sample, is indicative of the absence of endometrial cancer.

2. An in-vitro method comprising assessing a test sample from a woman who is under 65 years of age for the level of adiponectin, wherein: i) the presence of a level of adiponectin that is equal to or less than a reference level is indicative of the presence of a risk of endometrial cancer; ii) the presence of a level of adiponectin that is greater than a reference level is indicative of the absence of a risk of endometrial cancer; iii) the presence of a level of adiponectin that is less than a control level, by an amount that is statistically significant, is indicative of the presence of a risk of endometrial cancer; iv) the presence of a level of adiponectin that is greater than a control level, by an amount that is statistically significant, or is equal to a control level, is indicative of the absence of a risk of endometrial cancer; v) the presence of a level of adiponectin that is less than a level of adiponectin in a comparable negative control sample, by an amount that is statistically significant, is indicative of the presence of a risk of endometrial cancer; or vi) the presence of a level of adiponectin that is greater than a level of adiponectin in a comparable negative control sample, by an amount that is statistically significant, or is equal to a level of adiponectin in a comparable negative control sample, is indicative of the absence of a risk of endometrial cancer.

3. An in-vitro method comprising assessing a test sample from an individual for the level of adiponectin, wherein: i) the presence of a level of adiponectin that is equal to or less than a reference level is indicative of the presence of the epithelial cancer; ii) the presence of a level of adiponectin that is greater than a reference level is indicative of the absence of the epithelial cancer; iii) the presence of a level of adiponectin that is less than a control level, by an amount that is statistically significant, is indicative of the presence of the epithelial cancer; iv) the presence of a level of adiponectin that is greater than a control level, by an amount that is statistically significant, or is equal to a control level, is indicative of the absence of the epithelial cancer; v) the presence of a level of adiponectin that is less than a level of adiponectin in a comparable negative control sample, by an amount that is statistically significant, is indicative of the presence of the epithelial cancer; or vi) presence of a level of adiponectin that is greater than a level of adiponectin in a comparable negative control sample, by an amount that is statistically significant, or is equal to a level of adiponectin in a comparable negative control sample, is indicative of the absence of the epithelial cancer.

4. An in-vitro method comprising assessing a test sample fiom an individual for the level of adiponectin, wherein: i) the presence of a level of adiponectin that is equal to or less than a reference level is indicative of the presence of a risk of the epithelial cancer; ii) the presence of a level of adiponectin that is greater than a reference level is indicative of the absence of a risk of the epithelial cancer; iii) the presence of a level of adiponectin that is less than a control level, by an amount that is statistically significant, is indicative of the presence of a risk of the epithelial cancer; iv) the presence of a level of adiponectin that is greater than a control level, by an amount that is statistically significant, or is equal to a control level, is indicative of the absence of a risk of the epithelial cancer; v) the presence of a level of adiponectin that is less than a level of adiponectin in a comparable negative control sample, by an amount that is statistically significant, is indicative of the presence of a risk of the epithelial cancer; or vi) the presence of a level of adiponectin that is greater than a level of adiponectin in a comparable negative control sample, by an amount that is statistically significant, or is equal to a level of adiponectin in a comparable negative control sample, is indicative of the absence of a risk of the epithelial cancer.

5. An in-vitro method comprising assessing a test sample from the individual for the level of adiponectin, wherein: i) the presence of a level of adiponectin that is equal to or less than a reference level is indicative of the presence of a risk of relapse the epithelial cancer; ii) the presence of a level of adiponectin that is greater than a reference level is indicative of the absence of a risk of relapse of the epithelial cancer; iii) the presence of a level of adiponectin that is less than a control level, by an amount that is statistically significant, is indicative of the presence of a risk of relapse of the epithelial cancer; iv) a test sample from the individual for the level of adiponectin, wherein the presence of a level of adiponectin that is greater than a control level, by an amount that is statistically significant, or is equal to a control level, is indicative of the absence of a risk of relapse of the epithelial cancer; v) the presence of a level of adiponectin that is less than a level of adiponectin in a comparable negative control sample, by an amount that is statistically significant, is indicative of the presence of a risk of relapse of the epithelial cancer; or vi) the presence of a level of adiponectin that is greater than a level of adiponectin in a comparable negative control sample, by an amount that is statistically significant, or is equal to a level of adiponectin in a comparable negative control sample, is indicative of the absence of a risk of relapse of the epithelial cancer.

6. The method of any one of Claims 3 to 5, wherein the epithelial cancer is selected from the group consisting of: leukemia, colon cancer, ovarian cancer, and prostate cancer.

7. The method of any one of Claims 3 to 5, wherein the epithelial cancer is breast cancer, and the individual is a postmenopausal woman.

**Patentansprüche**

1. In-vitro-Verfahren, bei dem eine Probe von einer Frau, die unter 65 Jahre alt ist, auf den Adiponektin-Gehalt untersucht wird, wobei:

i) das Vorhandensein eines Adiponektin-Gehalts, der gleich oder kleiner ist als ein Referenzgehalt, indikativ ist für das Vorhandensein von endometrialem Krebs;
ii) das Vorhandensein eines Adiponektin-Gehalts, der größer ist als ein Referenzgehalt, indikativ ist für das Fehlen von endometrialem Krebs;
iii) das Vorhandensein eines Adiponektin-Gehalts, der um einen Betrag, der statistisch signifikant ist, kleiner ist als ein Kontrollgehalt, indikativ ist für das Vorhandensein von endometrialem Krebs;
iv) das Vorhandensein eines Adiponektin-Gehalts, der um einen Betrag, der statistisch signifikant ist, größer ist als ein Kontrollgehalt, oder gleich einem Kontrollgehalt ist, indikativ ist für das Fehlen von endometrialem Krebs;
v) das Vorhandensein eines Adiponektin-Gehalts, der um einen Betrag, der statistisch signifikant ist, kleiner ist als ein Adiponektin-Gehalt in einer vergleichbaren negativen Kontrollprobe, indikativ für das Vorhandensein von endometrialem Krebs ist; oder

vi) das Vorhandensein eines Adiponektin-Gehalts, der um einen Betrag, der statistisch signifikant ist, größer ist als ein Adiponektin-Gehalt in einer vergleichbaren negativen Kontrollprobe, oder gleich ist einem Adiponektin-Gehalt in einer vergleichbaren negativen Kontrollprobe, indikativ ist für das Fehlen von endometrialem Krebs.

2. In-vitro-Verfahren, bei dem eine Probe von einer Frau, die unter 65 Jahre alt ist, auf den Adiponektin-Gehalt untersucht wird, wobei:

i) das Vorhandensein eines Adiponektion-Gehalts, der gleich oder kleiner ist als ein Referenzgehalt, indikativ ist für das Vorhandensein eines Risikos für endometrialen Krebs;

ii) das Vorhandensein eines Adiponektin-Gehalts, der größer ist als ein Refrenzgehalt, indikativ ist für das Fehlen eines Risikos für endometrialen Krebs,

iii) das Vorhandensein eines Adiponektin-Gehalts, der um einen Betrag, der statistisch signifikant ist, kleiner ist als ein Kontrollgehalt, indikativ ist für das Vorhandensein eines Risikos für endometrialen Krebs;

iv) das Vorhandensein eines Adiponektin-Gehalts, der um einen Betrag, der statistisch signifikant ist, größer ist als ein Kontrollgehalt, oder gleich ist einem Kontrollgehalt, indikativ ist für das Fehlen eines Risikos für endometrialen Krebs;

v) das Vorhandensein eines Adiponektin-Gehalts, der um einen Betrag, der statistisch signifikant ist, kleiner ist als ein Adiponektin-Gehalt in einer vergleichbaren negativen Kontrollprobe, indikativ ist für das Vorhandensein eines Risikos für endometrialen Krebs; oder vi) das Vorhandensein eines Adiponektin-Gehalts, der um einen Betrag, der statistisch signifikant ist, größer ist als ein Adiponektin-Gehalt in einer vergleichbaren negativen Kontrollprobe, oder gleich ist einem Adiponektin-Gehalt in einer vergleichbaren negativen Kontrollprobe, indikativ ist für das Fehlen eines Risikos für endometrialen Krebs.

3. In-vitro-Verfahren, bei dem eine Probe von einem Individuum auf den Adiponektin-Gehalt untersucht wird, wobei:

i) das Vorhandensein eines Adiponektin-Gehalts, der gleich oder kleiner ist als ein Referenzgehalt, indikativ ist für das Vorhandensein von epithelialem Krebs;

ii) das Vorhandensein eines Adiponektin-Wertes, der größer ist als ein Referenzgehalt, indikativ ist für das Fehlen von epithelialem Krebs;

iii) das Vorhandensein eines Adiponektin-Gehalts, der um einen Betrag, der statistisch signifikant ist, kleiner ist als ein Kontrollgehalt, indikativ ist für das Vorhandensein von epithelialem Krebs;

iv) das Vorhandensein eines Adiponektin-Gehalts, der um einen Betrag, der statistisch signifikant ist, größer ist als ein Kontrollgehalt oder gleich ist einem Kontrollgehalt, indikativ ist für das Fehlen von epithelialem Krebs;

v) das Vorhandensein eines Adiponektin-Gehalts, der um einen Betrag, der statistisch signifikant ist, kleiner ist als ein Adiponektin-Gehalt in einer vergleichbaren negativen Kontrollprobe, indikativ ist für das Vorhandensein von epithelialem Krebs; oder

vi) das Vorhandensein eines Adiponektin-Gehalts, der um einen Betrag, der statistisch signifikant ist, größer ist als ein Adiponektin-Gehalt in einer vergleichbaren negativen Kontrollprobe, oder gleich ist einem Adiponektin-Gehalt in einer vergleichbaren negativen Kontrollprobe, indikativ ist für das Fehlen von epithelialem Krebs.

4. In-vitro-Verfahren, bei dem eine Probe von einem Individuum auf den Adiponektin-Gehalt untersucht wird, wobei:

i) das Vorhandensein eines Adiponektin-Gehalts, der gleich oder kleiner ist als ein Referenzgehalt, indikativ ist für das Vorhandensein eines Risikos für epithelialen Krebs;

ii) das Vorhandensein eines Adiponektin-Gehalts, der größer ist als ein Referenzgehalt, indikativ ist für das Fehlen eines Risikos für epithelialen Krebs;

iii) das Vorhandensein eines Adiponektion-Gehalts, der um einen Betrag, der statistisch signifikant ist, kleiner ist als ein Kontrollgehalt, indikativ ist für das Vorhandensein eines Risikos für epithelialen Krebs;

iv) das Vorhandensein eines Adiponektion-Gehalts, der um einen Betrag, der statistisch signifikant ist, größer ist als ein Kontrollgehalt, oder gleich ist einem Kontrollgehalt, indikativ ist für das Fehlen eines Risikos für epithelialen Krebs;

v) das Vorhandensein eines Adiponektin-Gehalts, der um einen Betrag, der statistisch signifikant ist, kleiner ist als ein Adiponektin-Gehalt in einer vergleichbaren negativen Kontrollprobe, indikativ ist für das Vorhandensein eines Risikos für epithelialen Krebs; oder

vi) das Vorhandensein eines Adiponektin-Gehalts, der um einen Betrag, der statistisch signifikant ist, größer ist als ein Adiponektin-Gehalt in einer vergleichbaren negativen Kontrollprobe, oder gleich ist einem Adiponektin-Gehalt in einer vergleichbaren negativen Kontrollprobe, indikativ ist für das Fehlen eines Risikos für epithelialen Krebs.

**5.** In-vitro-Verfahren, bei dem eine Probe von einem Individuum auf den Adiponektin-Gehalt untersucht wird, wobei:

i) das Vorhandensein eines Adiponektin-Gehalts, der gleich oder kleiner ist als ein Referenzgehalt, indikativ ist für das Vorhandensein eines Risikos für einen Rückfall von epithelialem Krebs;

ii) das Vorhandensein eines Adiponektin-Gehalts, der größer ist als ein Referenzgehalt, indikativ ist für das Fehlen eines Risikos für einen Rückfall von epithelialem Krebs;

iii) das Vorhandensein eines Adiponektin-Gehalts, der um einen Betrag, der statistisch signifikant ist, kleiner ist als ein Kontrollgehalt, indikativ ist für das Vorhandensein eines Risikos für einen Rückfall von epithelialem Krebs;

iv) das Vorhandensein eines Adiponektin-Gehalts, der um einen Betrag, der statistisch signifikant ist, größer ist als ein Kontrollgehalt, oder gleich ist einem Kontrollgehalt, indikativ ist für das Fehlen eines Risikos für einen Rückfall von epithelialem Krebs;

v) das Vorhandensein eines Adiponektin-Gehalts, der um einen Betrag, der statistisch signifikant ist, kleiner ist als ein Adiponektin-Gehalt in einer vergleichbaren negativen Kontrollprobe, indikativ ist für das Vorhandensein eines Risikos für einen Rückfall von epithelialem Krebs; oder

vi) das Vorhandensein eines Adiponektin-Gehalts, der um einen Betrag, der statistisch signifikant ist, größer ist als ein Adiponektin-Gehalt in einer vergleichbaren negativen Kontrollprobe, oder gleich ist einem Adiponektin-Gehalt in einer vergleichbaren negativen Kontrollprobe, indikativ ist für das Fehlen eines Risikos für einen Rückfall von epithelialem Krebs.

**6.** Verfahren nach einem der Ansprüche 3 bis 5, bei dem der epitheliale Krebs aus der Gruppe von Leukämie, Colonkrebs, Eierstockkrebs und Prostatakrebs ist.

**7.** Verfahren nach einem der Ansprüche 3 bis 5, bei dem der epitheliale Krebs Brustkrebs ist und das Individuum eine Frau nach der Menopause ist.

**Revendications**

**1.** Procédé *in vitro,* comprenant l'évaluation d'un échantillon de test prélevé chez une femme âgée de moins de 65 ans pour déterminer le taux d'adiponectine, dans lequel : i) la présence d'un taux d'adiponectine qui est égal ou inférieur à un taux de référence est indicative de la présence d'un cancer de l'endomètre ; ii) la présence d'un taux d'adiponectine qui est supérieur à un taux de référence est indicative de l'absence de cancer de endomètre ; iii) la présence d'un taux d'adiponectine qui est inférieur d'une quantité statistiquement significative à un taux de contrôle est indicative de la présence d'un cancer de l'endomètre ; iv) la présence d'un taux d'adiponectine qui est supérieur d'une quantité statistiquement significative à un taux de contrôle ou qui est égal à un taux de contrôle est indicative de l'absence de cancer de endomètre ; v) la présence d'un taux d'adiponectine qui est inférieur d'une quantité statistiquement significative à un taux d'adiponectine dans un échantillon de contrôle négatif comparable est indicative de la présence d'un cancer de l'endomètre ; ou vi) la présence d'un taux d'adiponectine qui est supérieur d'une quantité statistiquement significative à un taux d'adiponectine dans un échantillon de contrôle négatif comparable ou qui est égal à un taux d'adiponectine dans un échantillon de contrôle négatif comparable est indicative de l'absence de cancer de l'endomètre.

**2.** Procédé *in vitro*, comprenant l'évaluation d'un échantillon de test prélevé chez une femme âgée de moins de 65 ans pour déterminer le taux d'adiponectine, dans lequel : i) la présence d'un taux d'adiponectine qui est égal ou inférieur à un taux de référence est indicative de la présence d'un risque de cancer de l'endomètre ; ii) la présence d'un taux d'adiponectine qui est supérieur à un taux de référence est indicative de l'absence de risque de cancer de l'endomètre ; iii) la présence d'un taux d'adiponectine qui est inférieur d'une quantité statistiquement significative à un taux de contrôle est indicative de la présence d'un risque de cancer de l'endomètre ; iv) la présence d'un taux d'adiponectine qui est supérieur d'une quantité statistiquement significative à un taux de contrôle ou qui est égal à un taux de contrôle est indicative de l'absence de risque de cancer de l'endomètre ; v) la présence d'un taux d'adiponectine qui est inférieur d'une quantité statistiquement significative à un taux d'adiponectine dans un échantillon de contrôle négatif comparable est indicative de la présence d'un risque de cancer de l'endomètre ; ou vi) la présence d'un taux d'adiponectine qui est supérieur d'une quantité statistiquement significative à un taux d'adiponectine dans un échantillon de contrôle négatif comparable ou qui est égal à un taux d'adiponectine dans un échantillon de contrôle négatif comparable est indicative de l'absence de risque de cancer de l'endomètre.

**3.** Procédé *in vitro*, comprenant l'évaluation d'un échantillon de test d'un individu pour déterminer le taux d'adiponectine, dans lequel ; i) la présence d'un taux d'adiponectine qui est égal ou inférieur à un taux de référence est indicative de la présence d'un cancer épithélial ; ii) la présence d'un taux d'adiponectine qui est supérieur à un taux de référence est indicative de l'absence de cancer épithélial ; iii) la présence d'un taux d'adiponectine qui est inférieur d'une quantité statistiquement significative à un taux de contrôle est indicative de la présence d'un cancer épithélial ; iv) la présence d'un taux d'adiponectine qui est supérieur d'une quantité statistiquement significative à un taux de contrôle ou qui est égal à un taux de contrôle est indicative de l'absence de cancer épithélial ; v) la présence d'un taux d'adiponectine qui est inférieur d'une quantité statistiquement significative à un taux d'adiponectine dans un échantillon de contrôle négatif comparable est indicative de la présence d'un cancer épithélial ; ou vi) la présence d'un taux d'adiponectine qui est supérieur d'une quantité statistiquement significative à un taux d'adiponectine dans un échantillon de contrôle négatif comparable ou qui est égal à un taux d'adiponectine dans un échantillon de contrôle négatif comparable est indicative de l'absence de cancer épithélial.

4. Procédé in *vitro*, comprenant l'évaluation d'un échantillon de test d'un individu pour déterminer le taux d'adiponectine, dans lequel : i) la présence d'un taux d'adiponectine qui est égal ou inférieur à un taux de référence est indicative de la présence d'un risque de cancer épithélial ; ii) la présence d'un taux d'adiponectine qui est supérieur à un taux de référence est indicative de l'absence de risque de cancer épithélial ; iii) la présence d'un taux d'adiponectine qui est inférieur d'une quantité statistiquement significative à un taux de contrôle est indicative de la présence d'un risque de cancer épithélial ; iv) la présence d'un taux d'adiponectine qui est supérieur d'une quantité statistiquement significative à un taux de contrôle ou qui est égal à un taux de contrôle est indicative de l'absence de risque de cancer épithélial ; v) la présence d'un taux d'adiponectine qui est inférieur d'une quantité statistiquement significative à un taux d'adiponectine dans un échantillon de contrôle négatif comparable est indicative de la présence d'un risque de cancer épithélial ; ou vi) la présence d'un taux d'adiponectine qui est supérieur d'une quantité statistiquement significative à un taux d'adiponectine dans un échantillon de contrôle négatif comparable ou qui est égal à un taux d'adiponectine dans un échantillon de contrôle négatif comparable est indicative de l'absence de risque de cancer épithélial.

5. Procédé *in vitro,* comprenant l'évaluation d'un échantillon de test de l'individu pour déterminer le taux d'adiponectine, dans lequel : i) la présence d'un taux d'adiponectine qui est égal ou inférieur à un taux de référence est indicative de la présence d'un risque de rechute d'un cancer épithélial ; ii) la présence d'un taux d'adiponectine qui est supérieur à un taux de référence est indicative de l'absence de risque de rechute d'un cancer épithélial ; iii) la présence d'un taux d'adiponectine qui est inférieur d'une quantité statistiquement significative à un taux de contrôle est indicative de la présence d'un risque de rechute d'un cancer épithélial ; iv) la présence d'un taux d'adiponectine qui est supérieur d'une quantité statistiquement significative à un taux de contrôle ou qui est égal à un taux de contrôle est indicative de l'absence de risque de rechute d'un cancer épithélial ; v) la présence d'un taux d'adiponectine qui est inférieur d'une quantité statistiquement significative à un taux d'adiponectine dans un échantillon de contrôle négatif comparable est indicative de la présence d'un risque de rechute d'un cancer épithélial ; ou vi) la présence d'un taux d'adiponectine qui est supérieur d'une quantité statistiquement significative à un taux d'adiponectine dans un échantillon de contrôle négatif comparable ou qui est égal à un taux d'adiponectine dans un échantillon de contrôle négatif comparable est indicative de l'absence de risque de rechute d'un cancer épithélial.

6. Procédé selon l'une quelconque des revendications 3 à 5, dans lequel le cancer épithélial est choisi dans le groupe constitué par la leucémie, le cancer du côlon, le cancer des ovaires et le cancer de la prostate.

7. Procédé selon l'une quelconque des revendications 3 à 5, dans lequel le cancer épithélial est le cancer du sein et l'individu est une femme ménopausée.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1365022 A

- WO 9902546 A

**Non-patent literature cited in the description**

- **Ziel, H.K. ; Finkle, W.D.** *New Engl. J. Med,* 1975, vol. 293 (23), 1167-1170
- **Jick, S.S. et al.** *Epidemiology,* 1993, vol. 4 (1), 20-24
- **Jick, S.S.** *Epidemiology,* 1993, vol. 4 (4), 384
- **Bilezikian, J.P.** *Journal of Women's Health,* 1994, vol. 3 (4), 273-282
- **van Leeuwen, F.E. et al.** *Lancet,* 1994, vol. 343 (8895), 448-452
- **Fisher, B. et al.** *Journal of the National Cancer Institute,* 1994, vol. 86 (7), 527-537
- **Rose, P.G.** *N. Engl. J. Med.,* 1996, vol. 335 (9), 640-649
- **Judd, H.L. et al.** *Obstet Gynecol,* 1992, vol. 59, 680-6
- **Deslypere, J.P.** *Metabolism,* 1995, vol. 44, 24-27
- **Carroll, K.K.** *Lipids,* 1998, vol. 33, 1055-1059
- **Parazzini, F. et al.** *Int J. Cancer,* 1999, vol. 81 (4), 539-42
- **Scherer, P.E. et al.** *J Biol Chem,* 1995, vol. 270, 26746-26749
- **Nakano, Y. et al.** *J Biochem,* 1996, vol. 120, 803-812
- **Hu, E. et al.** *J Biol Chem,* 1996, vol. 271, 10697-10703
- **Maeda, K. et al.** *Biochem Biophys Res Commun,* 1996, vol. 221, 286-289
- **Arita, Y. et al.** *Biochem Biophy Res Commun,* 1999, vol. 257, 79-83
- **Weyer, C. et al.** *J Clin Endocrinol Metab,* 2001, vol. 86, 1930-1935
- **Hotta, K. et al.** *Arterioscler Thromb Vasc Biol,* 2000
- **Hu, X.-B. et al.** *Acta Biochim. Biophys. Sin.,* 2003, vol. 35 (11), 1023-1028
- **Fruebis, J. et al.** *PNAS USA,* 2001, vol. 98 (4), 2005-2010
- **Tomas, E. et al.** *PNAS USA,* 2002, vol. 99 (25), 16309-16313
- **Gallup, D.G. ; Stock, R.J.** *Obstet Gynecol,* 1984, vol. 64, 417-20
- **Peterson, E.P.** *Obstet Gynecol,* 1968, vol. 31, 702-7

- **Gallup, D.G. ; Stock, R.J.** *Obstet Gynecol,* 1984
- **Evans-Metcalf, E.R. et al.** *Obstet Gynecol,* 1998, vol. 91, 349-354
- **Rutanen, E.M. et al.** *J Clin Endocrinol Metab,* 1993, vol. 77, 199-204
- **Nagamani, M. et al.** *J Clin Endocrinol Metab,* 1988, vol. 67, 144-148
- **Brinton, L.A. et al.** *Am J Obstet Gynecol,* 1992, vol. 167, 1317-1325
- **Weiderpass, E. et al.** *Cancer Causes Control,* 2000, vol. 11, 185-192
- **La Vecchia, C. et al.** *Br J Cancer,* 1994, vol. 70, 950-953
- **Lupulescu, A.P.** *Cancer Res,* 1985, vol. 45, 3288-95
- **Hueson, J.C. ; Legro, N.** *Cancer Res,* 1972, vol. 31, 226-32
- **Nagamani, M. ; Stuart, C.A.** *Am J Obstet Gynecol,* 1998, vol. 179 (1), 6-12
- **Sheets, E.E. et al.** *Am J Obstet Gynecol,* 1985, vol. 153, 60-5
- **Nagamani, M. et al.** *Am J Obstet Gynecol,* 1991, vol. 165, 1865-71
- **Surrey, E. et al.** *Proceedings of the Thirty-eighth Annual Meeting of the Society for Gynecologic Investigation,* 20 March 1991
- **Bermont, L. et al.** *J Clin Endocrinol Metab,* 2001, vol. 86 (1), 363-8
- **Mick, G.J. et al.** *Endocrinology,* 2002, vol. 143 (3), 948-53
- **Hotta, K. et al.** *Arterioscler Thromb Vasc Biol,* 2000, vol. 20, 1595-1599
- **Haque, W.A. et al.** *J. Clin. Endocrinol. Metab.,* 2002, vol. 87 (5), 2395-98
- **Peiris, A.N. et al.** *Acta Med. Scand. Suppl.,* 1999, vol. 723, 179-188
- **Fujioka, S. et al.** *Int. J. Obes.,* vol. 15, 853-859
- **Yamauchi, T. et al.** *Nature Med.,* 2001, vol. 7, 941-946
- **Franceschi, S. et al.** *Ann Epidemiol.,* 1995, vol. 5, 69-75

- **Petridou, E. et al.** *J. Clin. Endocrinol Metab.,* 2003, vol. 88, 993-7
- **Yannakoulia, M. et al.** *J. clin. Endocrinol. Metab.,* 2003, vol. 88, 1780-6
- **Evans-Metcalf, E.R. et al.** *Obstet Gynecol.,* 1998, vol. 91, 349-54
- **Parazzini, F. et al.** *Gynecol Oncol,* vol. 41, 1-16
- **Parazzini, F. et al.** *Gynecol Oncol,* 1991, vol. 41, 1-16
- **Kaaks, R. et al.** *Cancer Epidemiol Biomarkers Prev,* 2002, vol. 11, 1531-43
- **Kauffman, R.P. et al.** *Am J Obstet Gynecol,* 2002, vol. 187, 1362-9
- **Augustin, L.S. et al.** *Int J Cancer,* 2003, vol. 105, 404-7
- **Hankinson, S. et al.** *Lancet,* 1998, vol. 351, 1393-6
- **Stoll, B.A.** *Int J Obes Relat Metab Disord,* 2002, vol. 26, 747-53
- **Michels, K.B. et al.** *Diabetes Care,* 2003, vol. 26, 1752-8
- **Stoll, B.A.** *Eur J. Clin. Nutr,* vol. 54, 83-7
- **Cleary, M.P. ; Maihle, N.J.** *Proc. Soc. Exp. Biol. Med,* 1997, vol. 216, 28-42
- **Franceschi, S. et al.** *Int. J. Cancer,* 1996, vol. 67, 181-6
- **Franceschi, S. et al.** *Int J. Cancer,* 1996, vol. 67, 181-6
- **van den Brandt, P.A. et al.** *Am J Epidemiol.,* 2000, vol. 152, 514-27